(19) Europäisches Patentamt / European Patent Office / Office européen des brevets

(11) EP 2 428 576 B1

(12) EUROPÄISCHE PATENTSCHRIFT

(45) Veröffentlichungstag und Bekanntmachung des Hinweises auf die Patenterteilung:
**14.01.2015 Patentblatt 2015/03**

(51) Int Cl.:
***C12P 7/02*** *(2006.01)* ***C12N 9/02*** *(2006.01)*
***C12P 41/00*** *(2006.01)*

(21) Anmeldenummer: **11189708.8**

(22) Anmeldetag: **20.07.2006**

(54) **Oxidoreduktasen zur stereoselektiven Reduktion von Ketoverbindungen**

Oxidoreductases for stereo-selective reduction of keto compounds

Oxydoréductases destinées à la réduction stéréosélective de composés cétoniques

(84) Benannte Vertragsstaaten:
**AT BE BG CH CZ DE DK ES FI FR GB HU IE IT LI LU NL PL PT SE SI SK**

(30) Priorität: **27.07.2005 AT 12612005**

(43) Veröffentlichungstag der Anmeldung:
**14.03.2012 Patentblatt 2012/11**

(60) Teilanmeldung:
**13002126.4**
**13165017.8**

(62) Dokumentnummer(n) der früheren Anmeldung(en) nach Art. 76 EPÜ:
**06776315.1 / 1 907 556**

(73) Patentinhaber: **Cambrex IEP GmbH**
**65203 Wiesbaden (DE)**

(72) Erfinder:
- **Tschentscher, Anke**
  **65347 Eltville - Hattenheim (DE)**
- **Gupta, Antje**
  **65207 Wiesbaden (DE)**
- **Dupont, Maria**
  **50354 Hürth (DE)**

(74) Vertreter: **Plate, Jürgen et al**
**Plate Schweitzer Zounek**
**Patentanwälte**
**Rheingaustrasse 196**
**65203 Wiesbaden (DE)**

(56) Entgegenhaltungen:
**EP-A1- 1 179 595**
**WO-A-03/091423**
**WO-A2-2004/111083**
**JP-A- 2005 095 022**
**US-A1- 2004 157 305**

- **DATABASE UniProt [Online] 1. Mai 2000 (2000-05-01), "SubName: Full=Orf1, orf2, orf3, orf4, orf5 genes, ; SubName: Full=Oxidoreductase;", XP002667605, gefunden im EBI accession no. UNIPROT:Q9S5E6 Database accession no. Q9S5E6**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 428 576 B1

SEQUENCE LISTING

<110> IEP GmbH

<120> Oxidoreduktasen zur stereoselektiven Reduktion von Ketoverbindungen

<130> I 11029D

<140> A 1261/2005
<141> 2005-07-27

<160> 130

<170> PatentIn version 3.3

<210> 1
<211> 282
<212> PRT
<213> Rhodotorula mucilaginosa

<400> 1

Met Pro Ala Thr Leu Arg Leu Asp Lys Lys Val Ala Ile Ile Thr Gly
1 5 10 15

Gly Ala Ser Gly Ile Gly Leu Glu Ser Ala Leu Val Phe Ala Gly Glu
20 25 30

Gly Ala His Val Val Val Ala Asp Ile Asn Val Glu Ala Ala Asn Arg
35 40 45

Ala Val Glu Ile Ile Lys Thr Gln Val Gln Asp Ala Pro Lys Ala Ile
50 55 60

Ala Val Lys Cys Asp Val Ser Lys Glu Asp Asp Ile Lys Asn Leu Val
65 70 75 80

Ala Thr Ala Val Glu Thr Phe Gly Arg Leu Asp Val Met Phe Asn Asn
85 90 95

Ala Gly Ile Met His Pro Glu Asp Asp Asn Ala Leu Asn Thr Ser Glu
100 105 110

Arg Ile Trp Asp Leu Thr Met Asn Ile Asn Val Lys Gly Val Trp Trp
115 120 125

Gly Cys Lys Tyr Ala Ile Asp Ala Met Arg Lys Asn Pro Gly Gly Ser
130 135 140

Lys Gly Ser Ile Ile Asn Thr Ala Ser Phe Val Ala Ile Leu Gly Ala
145 150 155 160

Ala Thr Pro Gln Ile Ala Tyr Thr Ala Ser Lys Gly Ala Val Leu Ala
165 170 175

Met Thr Arg Glu Leu Ala Met Val His Ala Arg Glu Gly Ile Arg Ile
180 185 190

Asn Ser Leu Cys Pro Gly Pro Leu Lys Thr Glu Leu Leu Met Lys Phe
195 200 205

Leu Asp Thr Pro Glu Lys Lys Glu Arg Arg Met Val His Ile Pro Met
210 215 220

Gly Arg Phe Gly Glu Ala Val Glu Gln Ala Arg Ala Ala Ala Phe Leu
225 230 235 240

Ala Ser Asp Asp Ser Ser Phe Ile Thr Gly Thr Asp Phe Lys Val Asp
245 250 255

Gly Gly Ile Ser Ser Cys Tyr Val Thr Pro Glu Gly Glu Gln Ala Leu
260 265 270

Ala Ala Pro Ser Asn Leu Ala Pro Lys Ala
275 280

<210> 2
<211> 254
<212> PRT
<213> Pichia farinosa

<400> 2

Met Ala Tyr Asn Phe Thr Asn Lys Val Ala Ile Ile Thr Gly Gly Ile
1 5 10 15

Ser Gly Ile Gly Leu Ala Thr Val Glu Lys Phe Ala Lys Leu Gly Ala
20 25 30

Lys Val Val Ile Gly Asp Ile Gln Lys Glu Glu Tyr Lys Glu Ala Ala
35 40 45

Phe Thr Asn Leu Lys Asn Lys Gly Ile Asn Leu Asp Gln Leu Thr Tyr
50 55 60

Val His Thr Asp Val Thr Ala Asn Ser Ala Asn Glu Asn Leu Leu Lys
65 70 75 80

Thr Ala Ile Ser Ser Phe Gly Gly Val Asp Phe Val Val Ala Asn Ser
85 90 95

Gly Ile Ala Lys Asp Gln Arg Ser Glu Glu Met Thr Tyr Glu Asp Phe
100 105 110

Lys Lys Ile Ile Asp Val Asn Leu Asn Gly Val Phe Ser Leu Asp Lys
115 120 125

Leu Ala Ile Asp Tyr Trp Leu Lys Asn Lys Lys Lys Gly Ser Ile Val
130 135 140

Asn Thr Gly Ser Ile Leu Ser Phe Val Gly Thr Pro Gly Leu Ser His
145 150 155 160

Tyr Cys Ala Ser Lys Gly Gly Val Lys Leu Leu Thr Gln Thr Leu Ala
165 170 175

Leu Glu Gln Ala Lys Asn Gly Ile Arg Val Asn Cys Ile Asn Pro Gly
180 185 190

Tyr Ile Arg Thr Pro Leu Leu Glu Phe Leu Pro Lys Asp Lys Tyr Asp
195 200 205

Ala Leu Val Asp Leu His Pro Met Gly Arg Leu Gly Glu Pro Glu Glu
210 215 220

Ile Ala Asn Ala Ile Ala Phe Leu Val Ser Asp Glu Ala Ser Phe Ile
225 230 235 240

Thr Gly Thr Thr Leu Leu Val Asp Gly Gly Tyr Thr Ala Gln
245 250

<210> 3
<211> 336
<212> PRT
<213> Pichia stipitis

<400> 3

```
Met Ser Ile Pro Ala Thr Gln Tyr Gly Phe Val Phe Thr Lys Lys Asp
1               5                   10                  15

Gly Leu Lys Ile Arg Glu Asn Met Pro Val Leu Glu Pro Lys Ala Asp
            20                  25                  30

Gln Val Leu Leu Lys Val Asp Ala Val Gly Leu Cys His Ser Asp Leu
        35                  40                  45

His Ala Ile Tyr Asp Gly Phe Asp Phe Gly Asp Asn Tyr Val Met Gly
    50                  55                  60

His Glu Ile Ala Gly Thr Ile Val Lys Lys Gly Ala Met Val Asp Phe
65                  70                  75                  80

Trp Asp Leu Asn Thr Arg Val Ala Cys Phe Gly Pro Asn Ser Cys Gly
                85                  90                  95
```

```
His Cys Gln Leu Cys Arg Thr Gly Phe Glu Asn Asp Cys Ile Asn Val
            100                 105                 110

Val Asn Gly Trp Phe Gly Leu Gly Lys Asn Gly Gly Tyr Gln Gln Tyr
        115                 120                 125

Leu Leu Val Glu Lys Pro Arg Asn Leu Val Ala Ile Pro Asp Asn Val
    130                 135                 140

Glu Leu Ser Asp Ala Ala Ala Ile Thr Asp Ala Leu Leu Thr Pro Tyr
145                 150                 155                 160

His Ala Met Arg Leu Ala Gly Val Arg Ser Gly Thr Lys Leu Leu Gln
                165                 170                 175

Ile Gly Ala Gly Gly Leu Gly Val Asn Gly Ile Gln Ile Ala Lys Ala
            180                 185                 190

Phe Gly Ala Gln Val Thr Val Ile Asp Lys Lys Pro Glu Ala Val Asp
        195                 200                 205

Val Ala Lys Ser Leu Gly Ala Asp Glu Val Tyr Ser Ala Leu Pro Glu
    210                 215                 220

Ser Thr Ser Pro Gly Ser Phe Asp Val Ala Ile Asp Tyr Val Ser Thr
225                 230                 235                 240

Gln Gly Thr Phe Asp Thr Cys Gln Lys Tyr Val Arg Ser Lys Gly Asn
                245                 250                 255

Ile Val Pro Val Gly Leu Ala Ala Pro Arg Ile Ser Phe Asn Leu Gly
            260                 265                 270

Asp Leu Ala Leu Arg Glu Ile Asn Val Leu Gly Ser Phe Trp Gly Thr
        275                 280                 285

Ser Ser Asp Leu Lys Glu Cys Phe Asp Leu Val Ser Lys Gly Lys Val
    290                 295                 300

Lys Pro Lys Val Thr Val Ala Pro Leu Lys Gln Leu Pro Glu Tyr Ile
305                 310                 315                 320

Val Lys Leu Gln Asn Ser Ala Tyr Glu Gly Arg Val Val Phe Lys Pro
                325                 330                 335
```

<210> 4
<211> 252
<212> PRT
<213> Leuconostoc carnosum

<400> 4

```
Met Thr Asp Arg Leu Lys Asn Lys Val Ala Ile Ile Thr Gly Gly Thr
1               5                   10                  15

Leu Gly Ile Gly Leu Ala Met Ala Gln Lys Phe Val Glu Glu Gly Ala
            20                  25                  30

Lys Val Val Ile Thr Gly Arg Arg Ala Asn Val Gly Ala Glu Ala Leu
        35                  40                  45

Lys Thr Ile Gly Asp Glu Ser Val Ala Arg Phe Val Gln His Asp Ala
    50                  55                  60

Ser Asp Glu Lys Gly Trp Ile Asp Leu Phe Glu Asn Thr Ile Lys Trp
65                  70                  75                  80

Phe Gly His Val Asp Thr Val Val Asn Asn Ala Gly Val Ala Ile Ala
                85                  90                  95

Lys Asn Ile Glu Glu Thr Thr Tyr Glu Asp Trp Lys Phe Leu Gln Ser
            100                 105                 110

Ile Asn Ser Asp Gly Val Phe Leu Gly Thr Lys Tyr Gly Met Gln Tyr
        115                 120                 125

Met Lys Asn Gln Ala Gly Gly Ala Ser Ile Ile Asn Met Ser Ser Ile
    130                 135                 140

Glu Gly Phe Val Gly Asp Pro Asn Leu Ala Ala Tyr Asn His Ser Lys
145                 150                 155                 160

Gly Gly Val Arg Ile Leu Ser Lys Ser Ala Ala Leu His Ala Ala Leu
                165                 170                 175

Asn Asp Tyr Asn Leu Arg Val Asn Thr Ile His Pro Gly Tyr Ile Lys
            180                 185                 190

Thr Pro Leu Val Asp Gly Ile Asp Gly Ala Glu Glu Ala Gln Ser Gln
        195                 200                 205

Arg Thr Gln Thr Pro Met Gly His Ile Gly Glu Pro Asn Asp Ile Ala
    210                 215                 220

Tyr Met Ala Val Tyr Leu Ala Ser Glu Glu Ser Lys Phe Ala Thr Gly
225                 230                 235                 240

Ala Glu Phe Val Val Asp Gly Gly Tyr Leu Ala Gln
```

245 250

<210> 5
<211> 347
<212> PRT
<213> Microbacterium sp.

<400> 5

```
Met Lys Ala Leu Gln Tyr Thr Lys Ile Gly Ser His Pro Glu Val Val
1               5                   10                  15

Glu Ile Glu Lys Pro Ser Pro Gly Pro Gly Gln Val Leu Leu Lys Val
            20              25                  30

Thr Ala Ala Gly Val Cys His Ser Asp Glu Phe Val Met Ser Leu Ser
        35                  40                  45

Glu Glu Gln Tyr Thr Ala Ala Gly Tyr Pro Leu Pro Leu Thr Leu Gly
    50                  55                  60

His Glu Gly Ala Gly Ile Val Glu Glu Leu Gly Glu Gly Val Glu His
65                  70                  75                  80

Leu Ser Val Gly Asp Ala Val Ala Val Tyr Gly Pro Trp Gly Cys Gly
                85                  90                  95

Arg Cys Arg Asn Cys Ala Gln Gly Lys Glu Asn Tyr Cys Thr Asn Ala
            100                 105                 110

Gln Ala Glu Gly Ile Met Pro Pro Gly Leu Gly Ala Pro Gly Ser Met
        115                 120                 125

Ala Glu Tyr Met Ile Val Asp Ser Ala Arg His Leu Val Pro Leu Gly
    130                 135                 140

Asp Leu Asp Pro Val Gln Asn Val Ser Leu Thr Asp Ala Gly Leu Thr
145                 150                 155                 160

Pro Tyr His Ala Val Lys Thr Ser Leu Pro Lys Leu Gly Ala Gly Thr
                165                 170                 175

Thr Ala Val Val Ile Gly Thr Gly Gly Leu Gly His Val Ala Ile Gln
            180                 185                 190

Ile Leu Arg Ala Val Ser Ala Ala Thr Val Ile Ala Leu Asp Val Asn
        195                 200                 205

Asp Glu Lys Leu Ala Leu Ala Lys Glu Val Gly Ala His His Thr Val
    210                 215                 220
```

Met Ser Asp Gly Gly Ala Val Asp Ala Ile Arg Arg Leu Thr Asp Gly
225 230 235 240

Leu Gly Ala Asn Ala Val Phe Asp Phe Val Gly Ala Asp Pro Thr Ile
245 250 255

Ala Thr Ala Ile Gly Ala Ala Ala Leu Asp Ala Asp Ile Thr Ile Val
260 265 270

Gly Ile Gly Gly Gly Thr Ala His Val Gly Phe Gly Thr Val Ala Tyr
275 280 285

Asp Ala Ala Leu Arg Ile Pro Tyr Trp Gly Ser Arg Ser Glu Leu Ile
290 295 300

Glu Val Leu Asp Leu Ala Arg Ser Gly Gln Val Gly Val Glu Ile Gln
305 310 315 320

Arg Tyr Ser Leu Asp Asp Gly Pro Lys Ala Tyr Glu Ala Leu Ala Ala
325 330 335

Gly Thr Val Arg Gly Arg Ala Val Ile Val Pro
340 345

<210> 6
<211> 347
<212> PRT
<213> Gordona rubropertinctus

<400> 6

Met Lys Ala Ile Gln Ile Ile Gln Pro Gly Lys Pro Pro Glu Leu Arg
1 5 10 15

Glu Val Glu Lys Pro Thr Pro Arg Pro Gly Gln Val Leu Leu Lys Val
20 25 30

Thr Ala Ala Gly Ala Cys His Ser Asp Asp Phe Val Leu Asn Leu Pro
35 40 45

Glu Glu Gly Phe Pro Tyr Pro Leu Pro Met Thr Leu Gly His Glu Gly
50 55 60

Ala Gly Val Val Ala Glu Val Gly Thr Gly Val Thr Gly Ile Ser Glu
65 70 75 80

Gly Thr Ser Val Ala Val Tyr Gly Ala Trp Gly Cys Gly Val Cys His
85 90 95

Phe Cys Ala Arg Gly Leu Glu Asn Tyr Cys Ser Arg Ala Gly Glu Leu

```
            100                 105                 110

Gly Ile Thr Pro Pro Gly Leu Gly Asn Pro Gly Ala Met Ala Glu Tyr
        115                 120                 125

Leu Leu Val Asp Asp Ala Arg His Leu Val Pro Leu Gly Asp Leu Asp
    130                 135                 140

Pro Val Ala Ala Val Pro Leu Thr Asp Ala Gly Leu Thr Pro Tyr His
145                 150                 155                 160

Ala Ile Lys Pro Ser Leu Pro Lys Leu Val Gly Gly Thr Thr Ala Val
                165                 170                 175

Val Ile Gly Ala Gly Gly Leu Gly His Val Gly Ile Gln Leu Leu Arg
            180                 185                 190

His Leu Thr Pro Ser Arg Val Ile Ala Leu Asp Val Ser Asp Asp Lys
        195                 200                 205

Leu Ala Phe Ala Arg Glu Val Gly Ala His Glu Val Val Leu Ser Asp
    210                 215                 220

Ala Asp Ala Val Ala Asn Val Arg Lys Ile Thr Gly Asn Asp Gly Ala
225                 230                 235                 240

Thr Ala Val Phe Asp Phe Val Gly Leu Gln Pro Thr Leu Asp Ile Ala
                245                 250                 255

Met Gly Val Val Gly Thr Met Gly Asp Val Val Ile Val Gly Ile Gly
            260                 265                 270

Asp Met Val Ala Thr Ala Lys Val Gly Phe Phe Thr Gln Pro Tyr Glu
        275                 280                 285

Val Ser Val Arg Ala Pro Tyr Trp Gly Ala Arg Asp Glu Leu Ile Glu
    290                 295                 300

Val Leu Asp Leu Ala Arg Asp Gly Val Leu Glu Val Ala Val Glu Arg
305                 310                 315                 320

Phe Ser Leu Asp Asp Gly Val Glu Ala Tyr Arg Arg Leu Ala Ala Asn
                325                 330                 335

Asp Leu Arg Gly Arg Ala Val Val Val Pro Asp
            340                 345
```

<210> 7
<211> 339

<212> PRT
<213> Pichia trehalophila

<400> 7

Met Cys Thr Ser Gln Ser Gly Tyr Val Tyr His Ser Gly Arg Pro Leu
1               5                   10                  15

Leu Thr Lys Glu Glu Leu Ser Ile Pro Glu Pro Lys Gly Ser Glu Ile
            20                  25                  30

Val Leu Lys Val Arg Ala Ala Gly Leu Cys Ser Ser Asp Val His Val
        35                  40                  45

Leu Asn Ser Ser Leu Pro Leu Thr Tyr Pro Asn Ser Phe Ala Met Gly
    50                  55                  60

His Glu Ile Ala Gly Glu Ile Tyr Lys Leu Gly Pro Asn Val Asp Ala
65                  70                  75                  80

Asp Lys Tyr Ser Ile Gly Asp Gly Tyr Ala Val His Gly Leu Asn Ser
                85                  90                  95

Cys Gly Asp Cys Ser Phe Cys Lys Val Gly Ser Gln Asn Leu Cys Thr
            100                 105                 110

Asp Asn Asn Ser Thr Trp Tyr Gly Leu Gly Lys Asn Gly Gly Tyr Glu
        115                 120                 125

Gln Tyr Val Leu Val Lys Ser Val His Asp Leu Ile Lys Ile Pro Glu
    130                 135                 140

Gly Val Ser Phe Ser Glu Ala Ala Val Ala Ser Asp Ala Val Leu Thr
145                 150                 155                 160

Pro Tyr His Ala Ile Ser Thr Cys Asn Leu Lys Ala Thr Ser Lys Val
                165                 170                 175

Leu Val Ile Gly Cys Gly Gly Leu Gly Thr Cys Ala Leu Gln Ile Ile
            180                 185                 190

Lys Leu Tyr Ser Ala Tyr Val Val Cys Val Asp Ser Lys Ala Glu Leu
        195                 200                 205

Glu Glu Leu Ala Lys Glu Tyr Gly Ala Asp Glu Phe Tyr Thr Asp Leu
    210                 215                 220

Ser Lys Ser Ser Val Pro Lys Met Ser Phe Asp Cys Val Phe Asp Phe
225                 230                 235                 240

Val Ala Ile Gln Pro Thr Phe Thr Ile Ser Gln Asn Tyr Val Lys Ser
245 250 255

Gly Gly Ile Ile Lys Pro Val Gly Leu Gly Ala Pro Ser Leu Thr Phe
260 265 270

Ser Leu Leu Asp Leu Gly Cys Arg Asp Val Lys Ile Ile Gly Ser Phe
275 280 285

Trp Gly Thr Gln Ala Glu Gln Lys Asp Cys Met Glu Leu Ile Gln Arg
290 295 300

Gly Leu Val Lys Pro Leu Ile Thr Ser Phe Thr Phe Asp Glu Phe Pro
305 310 315 320

Gln Ala Tyr Glu Leu Leu Ser Thr Gly Lys Ser Lys Gly Arg Leu Val
325 330 335

Ile Ser Gln

<210> 8
<211> 254
<212> PRT
<213> Candida nemodendra

<400> 8

Met Gly Tyr Asn Leu Ile Asn Lys Val Ala Val Val Thr Gly Gly Cys
1 5 10 15

Ser Gly Ile Gly Leu Ala Val Thr Lys Lys Tyr Leu Glu Leu Gly Ala
20 25 30

Lys Val Val Ile Gly Asp Val Ser Thr Lys Glu Lys Phe Asn Glu Val
35 40 45

Ser Ser Glu Leu Lys Val Ala Gly Leu Asn Val Asn Asn Leu Asn Phe
50 55 60

Val Ser Ala Asp Ser Ser Lys Glu Asp Asp Asn Lys Arg Leu Val Asp
65 70 75 80

Glu Ala Ile Lys Asn Phe Gly Gly Leu Asp Ile Val Cys Ala Asn Ala
85 90 95

Gly Ile Gly Ser Met Ile Pro Phe His Glu Met Thr Phe Glu Ala Trp
100 105 110

Arg Lys Leu Leu Ala Val Asn Leu Asp Gly Val Phe Leu Leu Asp Arg
115 120 125

```
Phe Ala Ile Asp Tyr Trp Leu Lys Asn Ser Lys Pro Gly Val Ile Val
    130                 135                 140

Asn Met Gly Ser Ile His Ser Phe Val Ala Ala Pro Gly Leu Ala His
145                 150                 155                 160

Tyr Ser Ala Ser Lys Gly Gly Val Lys Leu Leu Thr Glu Ala Leu Ala
                165                 170                 175

Leu Glu Tyr Ser Ser Lys Gly Ile Arg Val Asn Ser Val Asn Pro Ala
            180                 185                 190

Tyr Ile Gln Thr Ser Leu Leu Glu Phe Leu Pro Glu Asp Lys Met Asn
        195                 200                 205

Ala Leu Lys Ala Val His Pro Ile Gly Arg Leu Gly Lys Pro Glu Glu
    210                 215                 220

Val Ala Asn Ala Val Ala Phe Leu Ser Ser Asp Glu Ala Thr Phe Ile
225                 230                 235                 240

His Gly Thr Ser Leu Leu Val Asp Gly Gly Tyr Thr Ala Gln
                245                 250
```

<210> 9
<211> 849
<212> DNA
<213> Rhodotorula mucilaginosa

<220>
<221> gene
<222> (1) .. (849)

<400> 9

```
atgcctgcca ctctccgcct cgacaagaag gttgccatca tcaccggagg agcatccggg      60
atcggcctcg agtcggccct cgtctttgcc ggagaaggcg cccacgtcgt cgtcgccgac     120
atcaacgtcg aagctgccaa ccgcgccgtc gagatcatca agacccaggt tcaggacgcc     180
cccaaggcga tcgccgtcaa gtgcgacgtc tccaaggagg acgacatcaa gaacctcgtc     240
gcgactgctg tcgaaacttt tggcaggctc gatgtcatgt tcaacaacgc cggcatcatg     300
cacccccgagg acgacaatgc gctcaacacc tcggagcgca tctgggacct gaccatgaac    360
attaacgtca agggagtctg gtggggctgc aagtacgcga tcgacgccat gcgcaagaac     420
ccgggcggca gcaaggggag catcatcaac acggcttcgt tcgtcgccat cctcggagcg     480
gcgacgcctc agatcgcata caccgcttcg aagggtgccg tcttggccat gactcgcgag     540
ctcgccatgg ttcacgcgcg cgaagggatc cgaatcaact cgctctgccc cggtccgctc     600
```

aagacagagc tcctgatgaa gttcctcgac acgccggaga agaaggagcg ccggatggtg 660

cacatcccga tgggtcgctt cggtgaggcg gttgagcagg ctcgcgcggc cgcgttcctc 720

gctagcgacg acagcagctt catcaccgga actgacttca aggtcgacgg cggtatcagc 780

tcgtgctacg tcacgccgga gggcgagcag gccctcgcgg cgccgtccaa cttggctccc 840

aaggcgtag 849

<210> 10
<211> 765
<212> DNA
<213> Pichia farinosa

<220>
<221> gene
<222> (1)..(765)

<400> 10

atggcctata acttcactaa caaagtcgct atcattacag gaggaatttc cggtattggt 60

ttagctacag tcgagaaatt cgctaagttg ggtgctaaag tcgtcatagg agacattcaa 120

aaagaagaat ataaagaagc tgcttttaca aatttgaaga acaaaggaat taatcttgat 180

caattgacgt atgtccacac ggacgtcacc gcaaattcgg caaatgagaa ccttttaaag 240

actgctataa gctcctttgg tggcgttgac tttgtcgtag caaactctgg aatagcaaaa 300

gatcaacgtt ctgaagagat gacttatgaa gatttcaaga aaataatcga tgtcaactta 360

aacggtgttt tttccttgga taagctagca attgattatt ggttaaaaaa taagaaaaag 420

ggctctattg tcaacacggg atctattctt tcatttgttg gtacccccgg gttatcacat 480

tattgtgcgt caaagggtgg agtgaagtta ttgacacaaa ccttggctct cgaacaggct 540

aagaatggca taagagtgaa ttgtataaat cctggttata taagaacacc tttattagag 600

tttttgccta aggacaagta tgacgcttta gtggatcttc atccaatggg tagattaggt 660

gaacctgagg aaattgccaa tgccattgca ttcctcgtct ctgacgaagc gagcttcata 720

actggtacta ctctactcgt tgatggagga tatacagccc agtaa 765

<210> 11
<211> 1011
<212> DNA
<213> Pichia stipitis

<220>
<221> gene
<222> (1)..(1011)

<400> 11

```
atgtctattc ctgctacaca atatggtttc gtcttcacca aaaaggacgg tttaaaaatt      60
cgcgagaaca tgcctgttct cgaacccaag gctgaccaag tcttgcttaa agtcgacgca     120
gtaggattgt gtcactctga ccttcatgcc atctacgacg gcttcgactt tggtgacaat     180
tacgttatgg gccacgaaat cgccggcacc attgtcaaga agggagccat ggtcgacttt     240
tgggacctaa acacccgtgt tgcctgtttt ggtccaaact cctgtggcca ttgtcaactt     300
tgtcgtactg gttttgaaaa tgattgtatc aatgtcgtca acggctggtt tggattaggt     360
aaaaacggag gctaccagca atatttgttg gttgaaaagc ctcgtaattt ggttgctatt     420
ccagacaacg tcgagctgtc cgatgcagct gccattaccg acgctttgtt gaccccctac     480
catgccatga gattagctgg tgttagatca ggcacgaagc tcttgcaaat tggtgctgga     540
ggattgggag taaatggtat tcagattgct aaagcatttg gagctcaagt cactgttatc     600
gacaaaaagc ccgaggctgt agacgtcgct aagagcctag gcgcagatga agtatattct     660
gcacttcctg aatcaaccag tccgggaagt ttcgatgttg ctatcgacta cgtttctact     720
caaggcactt tcgacacttg tcaaaagtac gtcagatcta agggtaatat tgttcccgtt     780
ggattggccg ctccaagaat ttcgtttaac ttgggagatt tggcccttag agaaattaat     840
gtccttggta gcttctgggg tacatcatcc gacttgaagg aatgtttcga tttggtcagc     900
aagggcaaag tcaaacctaa ggtgactgtt gctccattga agcaattgcc tgaatacatt     960
gtcaagttac agaattcggc ctacgaaggt agagtcgtgt tcaagccatg a             1011
```

<210> 12
<211> 759
<212> DNA
<213> Leuconostoc carnosum

<220>
<221> gene
<222> (1) .. (759)

<400> 12

atgacagatc ggttaaagaa taaagttgct attatcactg gtggtacact tggtattggc 60

ttagcaatgg ctcaaaagtt tgtagaagaa ggcgctaaag ttgtcattac tgggcgtcgt 120

gctaatgttg gtgcagaagc gctaaagaca attggtgatg aatcagtagc acgatttgtt 180

caacatgatg catctgatga aaaaggctgg attgatttat ttgaaaatac gattaaatgg 240

tttggtcatg tcgatacggt tgtcaataat gccggtgttg caattgctaa aaacattgaa 300

gagacaacat atgaagactg gaaatttttg caatcaatca actctgatgg cgttttctta 360

ggaactaagt acggtatgca atatatgaaa aaccaagctg gtggtgcctc aattattaat 420

atgtcatcta ttgaaggatt tgttggtgat cctaacttag ctgcttataa tcattcaaaa 480

ggtggtgtcc gcattttgag taagtcagct gcactacatg cagcattgaa tgactataac 540

ttacgtgtca acacgattca cccaggatat atcaaaacac cattggttga tggtattgat 600

ggtgcagaag aagcccaatc acaaagaact caaacaccta tgggacatat tggtgaacct 660

aatgatattg catatatggc agtctattta gctagtgaag aatcaaagtt tgcaacaggt 720

gctgaattcg ttgttgatgg cggctatttg gcacaataa 759

<210> 13
<211> 1044
<212> DNA
<213> Microbacterium sp.

<220>
<221> gene
<222> (1)..(1044)

<400> 13

```
atgaaggcac tccagtacac gaagatcgga tcccaccccg aagtcgtcga gatcgagaag      60
ccctcgccgg gtcccgggca ggtactgctc aaagtcaccg ccgccggcgt ctgccactcg     120
gacgagttcg tgatgagcct cagcgaggag cagtacaccg ctgccggcta ccccctgccg     180
ctcaccctcg ggcacgaagg cgccggcatc gtcgaggagc tcggcgaagg tgtcgagcac     240
ctgagcgtcg gagacgccgt cgccgtctac ggcccctggg gttgcggccg ctgccgcaac     300
tgcgcgcagg gcaaggagaa ctactgcacg aacgcccagg cggaggggat catgcctccc     360
ggtctcgggg ctcccggctc aatggcggag tacatgatcg tcgacagcgc gcgacacctc     420
gttccgctcg gcgacctcga ccccgtgcag aacgtttcct tgacggatgc cggcctgacc     480
ccgtaccacg cggtcaagac gtcacttccg aagctgggcg ccggaacgac ggcggtcgtg     540
atcggcaccg gggtctcggg acacgtcgcg attcagatcc tgcgggcggt gtcggccgcg     600
accgtgatcg cgttggacgt caacgacgag aaactcgcgc tggccaagga ggtcggcgcc     660
catcacaccg tcatgagcga cggcggcgcc gtcgacgcga ttcgccggct caccgacggt     720
ctgggcgcga acgccgtctt cgacttcgtc ggtgcggacc cgacgatcgc gacggcgata     780
ggagcagccg cgctcgacgc agacatcacg atcgtcggca tcggcggcgg aacggctcac     840
gtcggtttcg gcaccgtcgc ttatgacgcg gcgcttcgca tcccgtattg ggctcgcgc      900
agcgaactga tcgaggtgct cgacctcgcg cgctcagggc aggtgggagt cgagatccag     960
cgctactcac tcgacgacgg cccgaaggcg tacgaggcgc tcgccgcggg cacggtccgc    1020
ggccgcgccg tcatcgtccc ctga                                           1044
```

<210> 14
<211> 1044
<212> DNA
<213> Gordona rubropertinctus

<220>
<221> gene
<222> (1)..(1044)

<400> 14

```
atgaaggcca ttcagatcat ccagccgggc aaaccgccgg agctgcgcga ggtcgagaaa      60
cccacgccgc gtcccgggca ggtgttgctg aaggtgacgg cagccggcgc ctgccattcg     120
```

```
gacgacttcg tcctcaacct gcccgaggaa ggattcccct atcccctgcc gatgacgctc   180
ggccacgaag gggccggcgt ggtcgccgag gtcggtaccg gcgtcaccgg catctccgag   240
ggcacctcgg tggccgtgta cggagcctgg ggttgcggcg tctgtcactt ctgcgcccgc   300
ggcctggaga actactgcag ccgagccggc gaactcggca tcaccccacc gggtctcggc   360
aacccgggcg cgatggccga gtacctgctc gtggacgacg cacggcatct ggtgccgctc   420
ggtgacctcg acccggtggc tgcagtccca ctcaccgatg ccggcctcac gccctaccac   480
gcgatcaaac cctcgcttcc gaagctggtc ggcggcacca cggcagtggt catcggagcc   540
ggtggtctcg ggcatgtcgg gatccaactg cttcgccacc tgaccccgtc ccgggtgatc   600
gctctcgacg tgagcgacga caagctcgcg ttcgcgcgcg aggtcggggc tcacgaggtg   660
gtgctctccg acgccgatgc cgtcgcgaac gtccgcaaga tcaccggcaa cgatggtgcg   720
accgccgtct tcgacttcgt cgggctgcaa cccacgctcg acatcgcgat gggcgtcgtc   780
gggaccatgg gtgacgtggt gatcgtgggc atcggtgaca tggtcgccac ggcgaaggtc   840
ggcttcttca cccagcccta cgaggtgtcg gtacgcgcgc cgtactgggg ggcgcgcgac   900
gaactcatcg aggtgctgga tctcgcacgc gatggggttc tcgaggtggc ggtcgaacga   960
ttctcactcg atgacggcgt cgaggcctac cggcgactgg ccgccaatga ccttcgaggg  1020
cgagcagtcg tggtgcctga ctga                                         1044
```

<210> 15
<211> 1020
<212> DNA
<213> Pichia trehalophila

<220>
<221> gene
<222> (1)..(1020)

<400> 15

```
atgtgtactt ctcaatctgg ctacgtttat cattctggta gaccactttt aactaaagaa    60
gaactttcaa ttcccgaacc aaaaggctct gaaattgttc taaaagttcg tgcagctggt   120
ttatgttcat cagatgttca tgttctaaac agcagtttac cattgactta cccaaacagt   180
tttgctatgg gtcatgaaat tgccggtgaa atttataagc ttggtccaaa cgttgacgct   240
gataaatatt caattggaga tggatatgca gttcatggtt tgaactcctg tggtgattgt   300
tccttctgta aggttggtag tcaaaacctg tgtaccgata acaattcaac ttggtacggt   360
ttaggtaaga atggtggtta tgaacagtac gttttagtta aaagtgttca tgacttaatt   420
aaaattccag aaggtgttag tttctcagag gccgcagttg cttcagatgc tgttttaact   480
ccatatcatg ctatcagcac ctgtaacttg aaggcaactt ccaaagtttt agttattggt   540
tgtggtgggt taggtacctg tgctttacaa atcatcaaat tgtacagtgc atatgttgtc   600
```

tgtgttgact ccaaagcaga attagaagaa cttgctaaag aatatggcgc tgatgaattc 660

tacaccgatt tatcaaaatc cagcgttccc aaaatgtcat ttgattgtgt ttttgatttt 720

gttgccattc agccaacttt caccatttct caaaattacg tcaagagcgg tggtatcatc 780

aaacctgttg gcttaggtgc tcctagctta acatttagtt tattggactt aggttgtaga 840

gacgttaaga tcattggctc tttctggggt acacaagctg aacaaaaaga ctgtatggaa 900

ctaattcaaa gaggtttagt caagccatta attacaagtt tcacttttga tgaatttcct 960

caagcttatg aattgttgtc gactgggaaa tccaagggta gattggttat cagtcaatag 1020

<210> 16
<211> 765
<212> DNA
<213> Candida nemodendra

<220>
<221> gene
<222> (1)..(765)

<400> 16

atgggttaca acttaatcaa caaagttgca gtcgtcacag gaggctgctc cggaattggt 60

ctcgcagtga ccaaaaaata tcttgaactg ggagcaaaag tggtcatagg agatgtatcc 120

actaaagaga agtttaacga ggtatcctcg gaactcaaag ttgcaggcct aaatgtaaac 180

aatttaaact ttgtttcagc agacagtagc aaagaagacg acaacaaacg tttagttgac 240

gaagcaatca agaactttgg tggtcttgat attgtgtgtg ctaatgccgg tatcggtagc 300

atgattccat tccatgaaat gacatttgaa gcatggagaa agttactcgc agtaaacctt 360

gatggtgtgt tcttgctaga cagattcgca attgattact ggttaaagaa tagcaaacct 420

ggtgttatcg tcaacatggg ttcaatccac tctttcgtcg ctgctccagg attagcacat 480

tactctgctt ccaagggagg tgtcaaacta ttgaccgaag ctcttgctct agagtactcg 540

tccaagggta ttagagtaaa ttctgtgaat cctgcatata ttcaaacctc attgctagaa 600

ttccttccag aagacaaaat gaatgccttg aaggcggtgc accctattgg ccgtttaggt 660

aaaccagaag aagtagccaa tgctgtcgca ttcctcagtt ccgatgaagc aaccttcata 720

catggtactt ctcttctagt tgatggaggt tacaccgctc aataa 765

<210> 17
<211> 9
<212> PRT
<213> Rhodotorula mucilaginosa

<220>
<221> PEPTIDE
<222> (1)..(9)

<400> 17

Met Pro Ala Thr Leu Arg Leu Asp Lys
1 5

<210> 18
<211> 13
<212> PRT
<213> Rhodotorula mucilaginosa

<220>
<221> PEPTIDE
<222> (1)..(13)

<400> 18

Gln Ala Leu Ala Ala Pro Ser Asn Leu Ala Pro Lys Ala
1 5 10

<210> 19
<211> 10
<212> PRT
<213> Rhodotorula mucilaginosa

<220>
<221> PEPTIDE
<222> (1)..(10)

<400> 19

Val Glu Ile Ile Lys Thr Gln Val Gln Asp
1 5 10

<210> 20
<211> 14
<212> PRT
<213> Rhodotorula mucilaginosa

<220>
<221> PEPTIDE
<222> (1)..(14)

<400> 20

Lys Val Ala Ile Ile Thr Gly Gly Ala Ser Gly Ile Gly Leu
1 5 10

<210> 21
<211> 8
<212> PRT
<213> Rhodotorula mucilaginosa

<220>
<221> PEPTIDE
<222> (1)..(8)

<400> 21

Ser Cys Tyr Val Thr Pro Glu Gly

1 5

<210> 22
<211> 8
<212> PRT
<213> Rhodotorula mucilaginosa

<220>
<221> PEPTIDE
<222> (1) .. (8)

<400> 22

Thr Asp Phe Lys Val Asp Gly Gly
1 5

<210> 23
<211> 10
<212> PRT
<213> Rhodotorula mucilaginosa

<220>
<221> PEPTIDE
<222> (1)..(10)

<400> 23

Val Met Phe Asn Asn Ala Gly Ile Met His
1 5 10

<210> 24
<211> 10
<212> PRT
<213> Rhodotorula mucilaginosa

<220>
<221> PEPTIDE
<222> (1) .. (10)

<400> 24

Val His Ala Arg Glu Gly Ile Arg Ile Asn
1 5 10

<210> 25
<211> 10
<212> PRT
<213> Pichia farinosa

<220>
<221> PEPTIDE
<222> (1) .. (10)

<400> 25

Met Ala Tyr Asn Phe Thr Asn Lys Val Ala
1 5 10

<210> 26
<211> 12
<212> PRT
<213> Pichia farinosa

<220>
<221> PEPTIDE
<222> (1)..(12)

<400> 26

```
Thr Thr Leu Leu Val Asp Gly Gly Tyr Thr Ala Gln
1               5                   10
```

<210> 27
<211> 9
<212> PRT
<213> Pichia farinosa

<220>
<221> PEPTIDE
<222> (1)..(9)

<400> 27

```
Glu Tyr Lys Glu Ala Ala Phe Thr Asn
1               5
```

<210> 28
<211> 16
<212> PRT
<213> Pichia farinosa

<220>
<221> PEPTIDE
<222> (1)..(16)

<400> 28

```
Asn Lys Val Ala Ile Ile Thr Gly Gly Ile Ser Gly Ile Gly Leu Ala
1               5                   10                  15
```

<210> 29
<211> 9
<212> PRT
<213> Pichia farinosa

<220>
<221> PEPTIDE
<222> (1) .. (9)

<400> 29

```
Asp Val Asn Leu Asn Gly Val Phe Ser
1               5
```

<210> 30
<211> 9

<212> PRT
<213> Pichia farinosa

<220>
<221> PEPTIDE
<222> (1)..(9)

<400> 30

```
His Tyr Cys Ala Ser Lys Gly Gly Val
1               5
```

<210> 31
<211> 8
<212> PRT
<213> Pichia farinosa

<220>
<221> PEPTIDE
<222> (1)..(8)

<400> 31

```
Asn Cys Ile Asn Pro Gly Tyr Ile
1               5
```

<210> 32
<211> 9
<212> PRT
<213> Pichia farinosa

<220>
<221> PEPTIDE
<222> (1) .. (9)

<400> 32

```
Leu His Pro Met Gly Arg Leu Gly Glu
1               5
```

<210> 33
<211> 11
<212> PRT
<213> Pichia stipitis

<220>
<221> PEPTIDE
<222> (1) ..(11)

<400> 33

```
Met Ser Ile Pro Ala Thr Gln Tyr Gly Phe Val
1               5                   10
```

<210> 34
<211> 11
<212> PRT
<213> Pichia stipitis

<220>
<221> PEPTIDE
<222> (1)..(11)

<400> 34

Ser Ala Tyr Glu Gly Arg Val Val Phe Lys Pro
1 5 10

<210> 35
<211> 9
<212> PRT
<213> Pichia stipitis

<220>
<221> PEPTIDE
<222> (1) .. (9)

<400> 35

Cys His Ser Asp Leu His Ala Ile Tyr
1 5

<210> 36
<211> 9
<212> PRT
<213> Pichia stipitis

<220>
<221> PEPTIDE
<222> (1)..(9)

<400> 36

Gly Tyr Gln Gln Tyr Leu Leu Val Glu
1 5

<210> 37
<211> 9
<212> PRT
<213> Pichia stipitis

<220>
<221> PEPTIDE
<222> (1) .. (9)

<400> 37

Thr Phe Asp Thr Cys Gln Lys Tyr Val
1 5

<210> 38
<211> 8
<212> PRT
<213> Pichia stipitis

<220>
<221> PEPTIDE

<222> (1)..(8)

<400> 38

Leu Leu Thr Pro Tyr His Ala Met
1 5

<210> 39
<211> 9
<212> PRT
<213> Pichia stipitis

<220>
<221> PEPTIDE
<222> (1)..(9)

<400> 39

Leu Val Ser Lys Gly Lys Val Lys Pro
1 5

<210> 40
<211> 9
<212> PRT
<213> Pichia stipitis

<220>
<221> PEPTIDE
<222> (1)..(9)

<400> 40

Gly Ala Gly Gly Leu Gly Val Asn Gly
1 5

<210> 41
<211> 10
<212> PRT
<213> Pichia stipitis

<220>
<221> PEPTIDE
<222> (1)..(10)

<400> 41

Ile Gln Ile Ala Lys Ala Phe Gly Ala Thr
1 5 10

<210> 42
<211> 8
<212> PRT
<213> Pichia stipitis

<220>
<221> PEPTIDE
<222> (1)..(8)

<400> 42

Leu Gly Ser Phe Trp Gly Thr Ser
1 5

<210> 43
<211> 10
<212> PRT
<213> Leuconostoc carnosum

<220>
<221> PEPTIDE
<222> (1)..(10)

<400> 43

Met Thr Asp Arg Leu Lys Asn Lys Val Ala
1 5 10

<210> 44
<211> 12
<212> PRT
<213> Leuconostoc carnosum

<220>
<221> PEPTIDE
<222> (1) .. (12)

<400> 44

Ala Glu Phe Val Val Asp Gly Gly Tyr Leu Ala Gln
1 5 10

<210> 45
<211> 9
<212> PRT
<213> Leuconostoc carnosum

<220>
<221> PEPTIDE
<222> (1)..(9)

<400> 45

Val Val Ile Thr Gly Arg Arg Ala Asn
1 5

<210> 46
<211> 9
<212> PRT
<213> Leuconostoc carnosum

<220>
<221> PEPTIDE
<222> (1) .. (9)

<400> 46

Gly Gly Ala Ser Ile Ile Asn Met Ser
1 5

<210> 47
<211> 8
<212> PRT
<213> Leuconostoc carnosum

<220>
<221> PEPTIDE
<222> (1) .. (8)

<400> 47

Thr Gln Thr Pro Met Gly His Ile
1 5

<210> 48
<211> 10
<212> PRT
<213> Leuconostoc carnosum

<220>
<221> PEPTIDE
<222> (1)..(10)

<400> 48

Gly Tyr Ile Lys Thr Pro Leu Val Asp Gly
1 5 10

<210> 49
<211> 11
<212> PRT
<213> Microbacterium sp.

<220>
<221> PEPTIDE
<222> (1) .. (11)

<400> 49

Met Lys Ala Leu Gln Tyr Thr Lys Ile Gly Ser
1 5 10

<210> 50
<211> 14
<212> PRT
<213> Microbacterium sp.

<220>
<221> PEPTIDE
<222> (1)..(14)

<400> 50

Leu Ala Ala Gly Thr Val Arg Gly Arg Ala Val Ile Val Pro
1 5 10

<210> 51
<211> 12
<212> PRT
<213> Microbacterium sp.

<220>
<221> PEPTIDE
<222> (1)..(12)

<400> 51

Cys His Ser Asp Glu Phe Val Met Ser Leu Ser Glu
1 5 10

<210> 52
<211> 10
<212> PRT
<213> Microbacterium sp.

<220>
<221> PEPTIDE
<222> (1)..(10)

<400> 52

Val Tyr Gly Pro Trp Gly Cys Gly Arg Cys
1 5 10

<210> 53
<211> 13
<212> PRT
<213> Microbacterium sp.

<220>
<221> PEPTIDE
<222> (1) .. (13)

<400> 53

Val Ser Leu Thr Asp Ala Gly Leu Thr Pro Tyr His Ala
1 5 10

<210> 54
<211> 12
<212> PRT
<213> Microbacterium sp.

<220>
<221> PEPTIDE
<222> (1)..(12)

<400> 54

Leu Arg Ala Val Ser Ala Ala Thr Val Ile Ala Leu
1 5 10

<210> 55
<211> 9

<212> PRT
<213> Microbacterium sp.

<220>
<221> PEPTIDE
<222> (1) .. (9)

<400> 55

**Asp Phe Val Gly Ala Asp Pro Thr Ile**
**1 5**

<210> 56
<211> 8
<212> PRT
<213> Gordona rubropertinctus

<220>
<221> PEPTIDE
<222> (1)..(8)

<400> 56

**Met Lys Ala Ile Gln Ile Ile Gln**
**1 5**

<210> 57
<211> 10
<212> PRT
<213> Gordona rubropertinctus

<220>
<221> PEPTIDE
<222> (1)..(10)

<400> 57

**Asp Leu Arg Gly Arg Ala Val Val Val Pro**
**1 5 10**

<210> 58
<211> 9
<212> PRT
<213> Gordona rubropertinctus

<220>
<221> PEPTIDE
<222> (1)..(9)

<400> 58

**Thr Ala Ala Gly Ala Cys His Ser Asp**
**1 5**

<210> 59
<211> 11
<212> PRT
<213> Gordona rubropertinctus

<220>
<221> PEPTIDE
<222> (1)..(11)

<400> 59

```
Thr Pro Tyr His Ala Ile Lys Pro Ser Leu Pro
1               5                   10
```

<210> 60
<211> 8
<212> PRT
<213> Gordona rubropertinctus

<220>
<221> PEPTIDE
<222> (1) .. (8)

<400> 60

```
Asp Phe Val Gly Leu Gln Pro Thr
1               5
```

<210> 61
<211> 8
<212> PRT
<213> Gordona rubropertinctus

<220>
<221> PEPTIDE
<222> (1)..(8)

<400> 61

```
Val Tyr Gly Ala Trp Gly Cys Gly
1               5
```

<210> 62
<211> 8
<212> PRT
<213> Gordona rubropertinctus

<220>
<221> PEPTIDE
<222> (1)..(8)

<400> 62

```
Asp Asp Ala Arg His Leu Val Pro
1               5
```

<210> 63
<211> 8
<212> PRT
<213> Gordona rubropertinctus

<220>
<221> PEPTIDE

<222> (1) .. (8)

<400> 63

Met Thr Leu Gly His Glu Gly Ala
1 5

<210> 64
<211> 14
<212> PRT
<213> Gordona rubropertinctus

<220>
<221> PEPTIDE
<222> (1)..(14)

<400> 64

Gly Gly Leu Gly His Val Gly Ile Gln Leu Leu Arg His Leu
1 5 10

<210> 65
<211> 18
<212> DNA
<213> Artificial

<220>
<223> synthetic construct

<220>
<221> misc_feature
<222> (1). (18)

<400> 65
caggaaacag ctatgacc 18

<210> 66
<211> 10
<212> PRT
<213> Rhodotorula mucilaginosa

<220>
<221> PEPTIDE
<222> (1)..(10)

<400> 66

Val Ala Thr Ala Val Glu Thr Phe Gly Arg
1 5 10

<210> 67
<211> 9
<212> PRT
<213> Rhodotorula mucilaginosa

<220>
<221> PEPTIDE
<222> (1)..(9)

<400> 67

Phe Gly Glu Ala Val Glu Gln Ala Arg
1 5

<210> 68
<211> 20
<212> DNA
<213> Artificial

<220>
<223> synthetic construct

<220>
<221> misc_feature
<222> (1) .. (20)

<400> 68
ccraaytcva cvgcvgtsgc 20

<210> 69
<211> 20
<212> DNA
<213> Artificial

<220>
<223> synthetic construct

<220>
<221> misc_feature
<222> (1)..(20)

<400> 69
gcctgytcga cvgcytcrcc 20

<210> 70
<211> 22
<212> DNA
<213> Artificial

<220>
<223> synthetic construct

<220>
<221> misc_feature
<222> (1) .. (22)

<400> 70
ctccgaggtg ttgagcgcat tg 22

<210> 71
<211> 25
<212> DNA
<213> Artificial

<220>
<223> synthetic construct

<220>
<221> misc_feature
<222> (1) .. (25)

<400> 71
gacgaggttc ttgatgtcgt cctcc 25

<210> 72
<211> 12
<212> PRT
<213> Pichia farinosa

<220>
<221> PEPTIDE
<222> (1)..(12)

<400> 72

Leu Leu Thr Gln Thr Leu Ala Leu Glu Gln Ala Lys
1 5 10

<210> 73
<211> 14
<212> PRT
<213> Pichia farinosa

<220>
<221> PEPTIDE
<222> (1)..(14)

<400> 73

Tyr Asn Phe Thr Asn Lys Val Ala Ile Ile Thr Gly Gly Ile
1 5 10

<210> 74
<211> 21
<212> DNA
<213> Artificial

<220>
<223> synthetic construct

<220>
<221> misc_feature
<222> (1) .. (21)

<220>
<221> misc_feature
<222> (10) .. (10)
<223> n is a, c, g, or t

<400> 74
ytgytcyaan gcyaadgtyt g 21

<210> 75
<211> 25
<212> DNA

<213> Artificial

<220>
<223> synthetic construct

<220>
<221> misc_feature
<222> (1) .. (25)

<220>
<221> misc_feature
<222> (11)..(11)
<223> n is a, c, g, or t

<400> 75
chaayaargt ngchathaty achgg 25

<210> 76
<211> 25
<212> DNA
<213> Artificial

<220>
<223> synthetic construct

<220>
<221> misc_feature
<222> (1) .. (25)

<400> 76
caacgttctg aagagatgac ttatg 25

<210> 77
<211> 20
<212> DNA
<213> Artificial

<220>
<223> synthetic construct

<220>
<221> misc_feature
<222> (1) .. (20)

<400> 77
ggtggagtga agttattgac 20

<210> 78
<211> 23
<212> DNA
<213> Artificial

<220>
<223> synthetic construct

<220>
<221> misc_feature
<222> (1) .. (23)

<400> 78
gccattctta gcctgttcga gag 23

<210> 79
<211> 25
<212> DNA
<213> Artificial

<220>
<223> synthetic construct

<220>
<221> misc_feature
<222> (1) .. (25)

<400> 79
gtcatctctt cagaacgttg atctt 25

<210> 80
<211> 7
<212> PRT
<213> Pichia stipitis

<220>
<221> PEPTIDE
<222> (1) .. (7)

<400> 80

Ala Asp Gln Val Leu Leu Lys
1 5

<210> 81
<211> 11
<212> PRT
<213> Pichia stipitis

<220>
<221> PEPTIDE
<222> (1)..(11)

<400> 81

Ile Ser Phe Asn Leu Gly Asp Leu Ala Leu Arg
1 5 10

<210> 82
<211> 21
<212> DNA
<213> Artificial

<220>
<223> synthetic construct

<220>
<221> misc_feature
<222> (1)..(21)

<220>
<221> misc_feature
<222> (12) .. (12)
<223> n is a, c, g, or t

<400> 82
gcygaycarg tnttrttraa r 21

<210> 83
<211> 21
<212> DNA
<213> Artificial

<220>
<223> synthetic construct

<220>
<221> misc_feature
<222> (1) .. (21)

<400> 83
ctyaargcya artcdccyaa r 21

<210> 84
<211> 20
<212> DNA
<213> Artificial

<220>
<223> synthetic construct

<220>
<221> misc_feature
<222> (1) .. (20)

<400> 84
ctaccatgcc atgagattag 20

<210> 85
<211> 20
<212> DNA
<213> Artificial

<220>
<223> synthetic construct

<220>
<221> misc_feature
<222> (1) .. (20)

<400> 85
gctgtagacg tcgctaagag 20

<210> 86
<211> 20
<212> DNA
<213> Artificial

<220>
<223> synthetic construct

<220>
<221> misc_feature
<222> (1) .. (20)

<400> 86
gattctcaag gctaagtcac 20

<210> 87
<211> 20
<212> DNA
<213> Artificial

<220>
<223> synthetic construct

<220>
<221> misc_feature
<222> (1) .. (20)

<400> 87
gatctaacac cagctaatct 20

<210> 88
<211> 22
<212> DNA
<213> Artificial

<220>
<223> synthetic construct

<220>
<221> misc_feature
<222> (1) .. (22)

<400> 88
ccaaaggagc ttatagcagt ct 22

<210> 89
<211> 29
<212> DNA
<213> Artificial

<220>
<223> synthetic construct

<220>
<221> misc_feature
<222> (1) .. (29)

<400> 89
gggaaattcc atatgcctgc cactctccg 29

<210> 90
<211> 33
<212> DNA

<213> Artificial

<220>
<223> synthetic construct

<220>
<221> misc_feature
<222> (1) .. (33)

<400> 90
cggcaagctt attacgcctt gggagccaag ttg 33

<210> 91
<211> 32
<212> DNA
<213> Artificial

<220>
<223> synthetic construct

<220>
<221> misc_feature
<222> (1) .. (32)

<400> 91
ggaaattcca tatggcctat aacttcacta ac 32

<210> 92
<211> 33
<212> DNA
<213> Artificial

<220>
<223> synthetic construct

<220>
<221> misc_feature
<222> (1)..(33)

<400> 92
cactgcatgc tgatggccta taacttcact aac 33

<210> 93
<211> 30
<212> DNA
<213> Artificial

<220>
<223> synthetic construct

<220>
<221> misc_feature
<222> (1) .. (30)

<400> 93
cgcaagctta ttactgggct gtatatcctc 30

<210> 94

<211> 33
<212> DNA
<213> Artificial

<220>
<223> synthetic construct

<220>
<221> misc_feature
<222> (1) .. (33)

<400> 94
ggaaattcca tatgatgtct attcctgcta cac 33

<210> 95
<211> 31
<212> DNA
<213> Artificial

<220>
<223> synthetic construct

<220>
<221> misc_feature
<222> (1) .. (31)

<400> 95
cactgcatgc gaatgtctat tcctgctaca c 31

<210> 96
<211> 33
<212> DNA
<213> Artificial

<220>
<223> synthetic construct

<220>
<221> misc_feature
<222> (1) .. (23)

<400> 96
cccaagctta tcatggtttg aacacgactc tac 33

<210> 97
<211> 10
<212> PRT
<213> Leuconostoc carnosum

<220>
<221> PEPTIDE
<222> (1)..(10)

<400> 97

```
Ile Glu Glu Thr Thr Tyr Glu Asp Trp Lys
1               5                   10
```

<210> 98
<211> 27
<212> DNA
<213> Artificial

<220>
<223> synthetic construct

<220>
<221> misc_feature
<222> (1) .. (27)

<220>
<221> misc_feature
<222> (13) .. (13)
<223> n is a, c, g, or t

<400> 98
gacagawmgw ttnaarggwa argthgc 27

<210> 99
<211> 29
<212> DNA
<213> Artificial

<220>
<223> synthetic construct

<220>
<221> misc_feature
<222> (1) .. (29)

<220>
<221> misc_feature
<222> (12) .. (12)
<223> n is a, c, g, or t

<400> 99
gcbgtrtawc cnccrtcdac dacraaytc 29

<210> 100
<211> 23
<212> DNA
<213> Artificial

<220>
<223> synthetic construct

<220>
<221> misc_feature
<222> (1)..(23)

<400> 100
ctaagccaat accaagtgta cca 23

<210> 101
<211> 27
<212> DNA

<213> Artificial

<220>
<223> synthetic construct

<220>
<221> misc_feature
<222> (1) .. (27)

<400> 101
gaacaaatcg tgctactgat tcatcac 27

<210> 102
<211> 25
<212> DNA
<213> Artificial

<220>
<223> synthetic construct

<220>
<221> misc_feature
<222> (1) .. (25)

<400> 102
gaagaagccc aatcacaaag aactc 25

<210> 103
<211> 23
<212> DNA
<213> Artificial

<220>
<223> synthetic construct

<220>
<221> misc_feature
<222> (1) .. (23)

<400> 103
ggcagtctat ttagctagtg aag 23

<210> 104
<211> 14
<212> PRT
<213> Microbacterium sp.

<220>
<221> PEPTIDE
<222> (1)..(14)

<400> 104

```
Met Lys Ala Leu Gln Tyr Thr Lys Ile Gly Ser His Pro Glu
1               5                   10
```

<210> 105
<211> 11

<212> PRT
<213> Microbacterium sp.

<220>
<221> PEPTIDE
<222> (1)..(11)

<400> 105

Ala Tyr Glu Ala Leu Ala Ala Gly Thr Val Val
1 5 10

<210> 106
<211> 20
<212> DNA
<213> Artificial

<220>
<223> synthetic construct

<220>
<221> misc_feature
<222> (1) .. (20)

<400> 106
ctscartaca cvaagatcgg 20

<210> 107
<211> 20
<212> DNA
<213> Artificial

<220>
<223> synthetic construct

<220>
<221> misc_feature
<222> (1) .. (20)

<400> 107
gcbgcsagbg cytcrtabgc 20

<210> 108
<211> 20
<212> DNA
<213> Artificial

<220>
<223> synthetic construct

<220>
<221> misc_feature
<222> (1) .. (20)

<400> 108
tcctcgctga ggctcatcac 20

<210> 109

<211> 22
<212> DNA
<213> Artificial

<220>
<223> synthetic construct

<220>
<221> misc_feature
<222> (1) .. (22)

<400> 109
gcttctcgat ctcgacgact tc 22

<210> 110
<211> 19
<212> DNA
<213> Artificial

<220>
<223> synthetic construct

<220>
<221> misc_feature
<222> (1)..(19)

<400> 110
gcgcagcgaa ctgatcgag 19

<210> 111
<211> 22
<212> DNA
<213> Artificial

<220>
<223> synthetic construct

<220>
<221> misc_feature
<222> (1) .. (22)

<400> 111
gatccagcgc tactcactcg ac 22

<210> 112
<211> 10
<212> PRT
<213> Gordona rubropertinctus

<220>
<221> PEPTIDE
<222> (1)..(10)

<400> 112

```
Met Lys Ala Ile Gln Ile Ile Gln Pro Gly
1               5                   10
```

<210> 113
<211> 13
<212> PRT
<213> Gordona rubropertinctus

<220>
<221> PEPTIDE
<222> (1)..(13)

<400> 113

```
Val Gly Phe Phe Thr Gln Pro Tyr Glu Val Ser Val Arg
1               5                   10
```

<210> 114
<211> 26
<212> DNA
<213> Artificial

<220>
<223> synthetic construct

<220>
<221> misc_feature
<222> (1) .. (26)

<220>
<221> misc_feature
<222> (9) .. (9)
<223> n is a, c, g, or t

<400> 114
atgaargcna tycaratyat ycarcc 26

<210> 115
<211> 22
<212> DNA
<213> Artificial

<220>
<223> synthetic construct

<220>
<221> misc_feature
<222> (1).. (22)

<220>
<221> misc_feature
<222> (8) .. (8)
<223> n is a, c, g, or t

<220>
<221> misc_feature
<222> (14) .. (14)
<223> n is a, c, g, or t

<400> 115
cytcrtangg ytgngtraar aa 22

<210> 116
<211> 21
<212> DNA
<213> Artificial

<220>
<223> synthetic construct

<220>
<221> misc_feature
<222> (1) .. (21)

<400> 116
gaggacgaag tcgtccgaat g 21

<210> 117
<211> 21
<212> DNA
<213> Artificial

<220>
<223> synthetic construct

<220>
<221> misc_feature
<222> (1)..(21)

<400> 117
gccgtcacct tcagcaacac c 21

<210> 118
<211> 21
<212> DNA
<213> Artificial

<220>
<223> synthetic construct

<220>
<221> misc_feature
<222> (1) .. (21)

<400> 118
ctcgacgtga gcgacgacaa g 21

<210> 119
<211> 21
<212> DNA
<213> Artificial

<220>
<223> synthetic construct

<220>
<221> misc_feature
<222> (1) .. (21)

<400> 119

gcaagatcac cggcaacgat g 21

<210> 120
<211> 37
<212> DNA
<213> Artificial

<220>
<223> synthetic construct

<220>
<221> misc_feature
<222> (1) .. (37)

<400> 120
catatggcta gcatgacaga tcggttaaag aataaag 37

<210> 121
<211> 35
<212> DNA
<213> Artificial

<220>
<223> synthetic construct

<220>
<221> misc_feature
<222> (1) .. (35)

<400> 121
gcgcggatcc atgacagatc ggttaaagaa taaag 35

<210> 122
<211> 29
<212> DNA
<213> Artificial

<220>
<223> synthetic construct

<220>
<221> misc_feature
<222> (1) .. (29)

<400> 122
cccaagcttc ttattgtgcc aaatagccg 29

<210> 123
<211> 31
<212> DNA
<213> Artificial

<220>
<223> synthetic construct

<220>
<221> misc_feature
<222> (1)..(31)

<400> 123
ggaaattcca tatgaaggca ctccagtaca c 31

<210> 124
<211> 32
<212> DNA
<213> Artificial

<220>
<223> synthetic construct

<220>
<221> misc_feature
<222> (1) .. (32)

<400> 124
cactgcatgc tgatgaaggc actccagtac ac 32

<210> 125
<211> 28
<212> DNA
<213> Artificial

<220>
<223> synthetic construct

<220>
<221> misc_feature
<222> (1) .. (28)

<400> 125
cccaagctta acgtcagggg acgatgac 28

<210> 126
<211> 33
<212> DNA
<213> Artificial

<220>
<223> synthetic construct

<220>
<221> misc_feature
<222> (1) .. (33)

<400> 126
cactgcatgc gaatgaaggc cattcagatc atc 33

<210> 127
<211> 33
<212> DNA
<213> Artificial

<220>
<223> synthetic construct

<220>
<221> misc_feature

<222> (1) .. (33)

<400> 127
cccaagctta ttagtcaggc accacgactg ctc 33

<210> 128
<211> 18
<212> DNA
<213> Artificial

<220>
<223> synthetic construct

<220>
<221> misc_feature
<222> (1) .. (18)

<400> 128
tgtaaaacga cggccagt 18

<210> 129
<211> 336
<212> PRT
<213> Lodderomyces elongisporus

<400> 129

```
Met Ser Ile Pro Thr Thr Gln Tyr Gly Phe Val Tyr Asn Lys Ser Ser
1               5                   10                  15

Gly Leu Thr Leu Asn Lys Ser Ile Pro Val Ala Ser Ala Gly Val Gly
```

20 25 30

Gln Leu Leu Met Lys Val Asp Ser Val Gly Leu Cys His Ser Asp Leu
35 40 45

His Val Ile Tyr Glu Gly Leu Asp Cys Gly Asp Asn Tyr Val Met Gly
50 55 60

His Glu Ile Ala Gly Thr Val Val Asp Val Gly Pro Glu Val Asp Arg
65 70 75 80

Trp Asn Val Gly Asp Arg Val Ala Ala Val Gly Pro Asn Gly Cys Gly
85 90 95

Gly Cys Arg Ala Cys Arg Asp Gly Ile Glu Asn Val Cys Lys His Ser
100 105 110

Phe Gly Asn Trp Tyr Gly Leu Gly Ser Asp Gly Gly Tyr Gln Gln Tyr
115 120 125

Leu Leu Val Gln Lys Pro Arg Asn Leu Val Lys Ile Pro Asp Asn Val
130 135 140

Pro Ser Asp Val Ala Ala Ala Ser Thr Asp Ala Val Leu Thr Pro Tyr
145 150 155 160

His Ala Ile Lys Met Ala Gly Val Gly Pro Thr Ser Lys Val Leu Ile
165 170 175

Val Gly Ala Gly Gly Leu Gly Cys Asn Ala Val Gln Val Ala Lys Ala
180 185 190

Phe Gly Ala His Val Thr Ile Leu Asp Lys Lys Glu Arg Ala Arg Ala
195 200 205

Glu Ala Val Lys Phe Gly Ala Asp Val Ala Tyr Glu Ser Leu Pro Leu
210 215 220

Ser Thr Glu Pro Gly Ser Phe Asp Ala Cys Leu Asp Phe Val Ser Val
225 230 235 240

Gln Ala Thr Phe Gly Ile Cys Gln Lys Phe Cys Ala Pro Lys Gly Cys
245 250 255

Ile Ile Pro Ala Gly Leu Gly Ala Pro Lys Leu Thr Leu Asp Leu Ala
260 265 270

Asp Leu Asp Leu Arg Glu Ile Arg Ile Leu Gly Thr Phe Trp Gly Thr
275 280 285

```
Ala Thr Asp Leu Glu Glu Val Phe Asp Leu Val Gly Lys Gly Leu Val
    290                 295                 300

Lys Pro Met Val Arg Ala Ala Lys Leu Glu Glu Leu Pro Asp Tyr Ile
305                 310                 315                 320

Glu Lys Leu Arg Lys Asn Glu Tyr Glu Gly Arg Ile Val Phe Asn Pro
                325                 330                 335
```

<210> 130
<211> 1012
<212> DNA
<213> Lodderomyces elongisporus

<220>
<221> gene
<222> (1)..(1011)

<400> 130

```
atgtcaattc caactaccca atatggtttt gtttacaata agtcgtctgg cttaacattg      60
aacaagagta tacctgttgc ctcggcaggt gtgggtcaat tgcttatgaa ggttgactct     120
gttggattgt gccactcgga cctccatgtg atttacgaag gtttggattg tggtgataac     180
tatgtcatgg gccatgagat tgccggtacc gtggttgatg ttggtccaga ggttgataga     240
tggaatgttg gtgatagagt tgccgctgtg ggtccaaatg gttgtggtgg ttgcagagcc     300
tgtcgcgacg gaattgaaaa tgtatgtaaa cactcttttg gtaattggta tggcttgggc     360
tcagatggcg gataccaaca atatttgctt gtgcaaaaac cacgcaattt ggttaagatt     420
cctgacaatg ttccttcgga tgtagctgca gcctcgactg acgctgtatt gacaccatac     480
cacgcaatca agatggctgg tgtggggcca acatcaaagg tgctcattgt tggcgctggt     540
ggcttgggct gcaatgccgt gcaagttgcc aaggcatttg gtgctcatgt cactattttg     600
gacaagaagg aacgcgcgcg cgctgaagct gtcaagtttg gtgccgacgt tgcctatgag     660
agcttaccac tgagcaccga gccaggctca tttgatgcat gtttggattt tgtttctgtg     720
caagcaacgt ttggcatttg ccaaaagttt tgtgcaccaa aaggttgcat catccccgcg     780
gggctcggtg caccaaagtt gacgcttgat ttggcagatt tggatttgcg cgaaattcgt     840
attttgggta ctttttgggg aaccgcgacc gatttggagg aggtgtttga cttggttgga     900
aagggacttg ttaagcccat ggtgcgtgca gccaagttgg aggaattgcc agactatatt     960
gaaaagttga gaaagaatga atatgaaggt agaattgtct ttaatccata ag            1012
```

## Beschreibung

Die vorliegende Erfindung betrifft ein Verfahren zur enantioselektiven enzymatischen Reduktion einer Ketoverbindung zur entsprechenden chiralen Hydroxyverbindung, wobei die Ketoverbindung mit einer Oxidoreduktase reduziert wird. Die Erfindung betrifft ferner neue Oxidoreduktasen zur Verwendung bei der enantioselektiven Reduktion von Ketoverbindungen zu chiralen Hydroxyverbindungen.

Optisch aktive Hydroxyverbindungen sind wertvolle chirale Bausteine mit breiter Anwendung für die Synthese von pharmakologisch wirksamen Verbindungen, aromatischen Substanzen, Pheromonen, Agrochemikalien und Enzyminhibitoren. Dabei ist insbesondere im Pharmasektor ein steigender Bedarf an chiralen Verbindungen und somit chiralen Synthesetechnologien zu verzeichnen, da racemische Verbindungen in Zukunft kaum noch als Arzneimittel Verwendung finden werden.

Die asymmetrische Reduktion prochiraler Ketoverbindungen ist ein Sektor der stereoselektiven Katalyse, in dem die Biokatalyse eine leistungsfähige Konkurrenztechnologie zur chemischen Katalyse darstellt. Die chemische asymmetrische Hydrierung erfordert den Einsatz hochgiftiger und umweltbelastender Schwermetallkatalysatoren, extremer und somit energieintensiver Reaktionsbedingungen sowie große Mengen organischer Lösungsmittel. Des weiteren sind diese Verfahren häufig gekennzeichnet durch Nebenreaktionen und unzureichende Enantiomerenüberschüsse.

Reduktionen prochiraler Ketoverbindungen zu Hydroxyverbindungen und umgekehrt kommen in der Natur in zahlreichen biochemischen Pathways, sowohl im Primärstoffwechsel als auch im Sekundärstoffwechsel, in jedem Organismus vor und werden von unterschiedlichen Typen von sekundären Alkoholdehydrogenasen und Oxidoreduktasen katalysiert. Diese Enzyme sind in der Regel cofaktorabhängig.

Die prinzipielle Machbarkeit der Nutzung von Biokatalysatoren zu Reduktion von prochiralen Ketoverbindungen zu chiralen Hydroxyverbindungen wurde in der Vergangenheit wiederholt anhand von Modellsystemen demonstriert, wobei sowohl mit isolierten Oxidoreduktasen als auch mit unterschiedlichen Ganzzellbiotransformationssystemen gearbeitet wurde. Dabei erwies sich der biokatalytische Ansatz im wesentlichen hinsichtlich der milden Reaktionsbedingungen, fehlender Nebenprodukte und oft wesentlich besseren erreichbaren Enantiomerenüberschüssen als vorteilhaft. Die Verwendung isolierter Enzyme ist gegenüber Verfahren mit ganzen Zellen hinsichtlich des erreichbaren Enantiomerenüberschuss, der Entstehung von Abbau- und Nebenprodukten als auch hinsichtlich der Produktisolation von Vorteil. Des weiteren ist die Verwendung von Ganzzellprozessen nicht jedem Chemieunternehmen möglich, da dafür spezielle Ausrüstung und Know-How erforderlich sind.

Jüngst konnte gezeigt werden, dass die Verwendung isolierter Oxidoreduktasen in wässrig/organischen Zwei -Phasensystemen mit organischen Lösungsmitteln äußerst effizient, ökonomisch und auch in hohen Konzentrationen (> 5%) möglich ist. Bei den beschriebenen Systemen bildet dabei die zu reduzierende, meist schlecht wasserlösliche Ketoverbindung zusammen mit dem organischen Lösungsmittel die organische Phase. Teilweise kann auch auf das organische Lösungsmittel selbst verzichtet werden, dann wird die organische Phase von der zu reduzierenden Ketoverbindung gebildet (DE 10119274, DE 10327454.4, DE 103 37 401.9, DE 103 00 335.5). Die Coenzymregenerierung wird dabei durch die gleichzeitige Oxidation sekundärer Alkohole realisiert, wobei in den meisten Fällen das preiswerte wassermischbare 2-Propanol verwendet wird.

Beispiele für geeignete R-und S-spezifische Oxidoreduktasen und Dehydrogenasen hoher Enantioselektivität sind:

*Carbonylreduktase aus Candida parapsilosis* (CPCR) (US 5,523,223 und US 5,763,236, (Enzyme Microb Technol. 1993 Nov;15(11):950-8)) und *Pichia capsulata* (DE10327454.4). *Carbonylreduktase aus Rhodococcus erythropolis* (RECR) (US 5,523,223), *Norcardia fusca* (Biosci. Biotechnol. Biochem.,63 (10) (1999), Seiten 1721-1729), (Appl Microbiol Biotechnol. 2003 Sep;62(4):380-6. Epub 2003 Apr 26), und *Rhodococcus ruber* (J Org Chem. 2003 Jan 24;68(2):402-6.).

R-spezifische sekundären Alkoholdehydrogenasen aus Organismen der Gattung *Lactobacillus* (Lactobacillus kefir (US5200335), Lactobacillus brevis (DE 19610984 A1) (Acta Crystallogr D Biol Crystallogr. 2000 Dec;56 Pt 12:1696-8), Lactobacillus minor (DE10119274) oder *Pseudomonas* (US 05385833)(Appl Microbiol Biotechnol. 2002 Aug;59(4-5):483-7. Epub 2002 Jun 26., J. Org. Chem. 1992, 57, 1532)

Die heute bekannten Enzyme reichen aber bei weitem nicht aus, um das gesamte Marktpotential an stereoselektiven Reduktionen von Ketoverbindungen auszuschöpfen. Das ist einerseits damit zu begründen, dass die einzelnen Enzyme über sehr unterschiedliche Eigenschaften bezüglich Substratspektrum, pH-Optima sowie Temperatur- und Lösungsmittelstabilitäten verfügen, die sich häufig gegenseitig ergänzen. Daher können selbst verhältnismäßig ähnliche, homologe Enzyme im Hinblick auf ein bestimmtes Substrat ein völlig unterschiedliches Umsetzungsverhalten aufweisen. Andererseits sind längst nicht alle beschriebenen Enzyme kloniert und in ausreichendem Maße überexprimierbar, was bedeutet, daß diese Enzyme für eine industrielle Anwendung nicht zur Verfügung stehen. Daher ist es zur möglichst breiten Ausschöpfung des synthetischen Potentials der enzymatischen asymmetrischen Hydrierung notwendig, über ein möglichst breites Portfolio industriell zugänglicher unterschiedlicher Oxidoreduktasen zu verfügen, die zudem für eine Anwendung in wässerig/organischen Zweiphasensystemen mit organischen Lösungsmitteln geeignet sind.

Gegenstand der vorliegenden Erfindung sind nun eine Reihe neuer, enantioselektiver, R-und S-spezifischer Oxidoreduktasen, die sich durch gute Stabilität in wässerig/organischen Zweiphasensystemen sowie durch gute Exprimierbarkeit in Escherichia coi (>500 Units/g E. coli Biofeuchtmasse) auszeichnen, sowie ein Verfahren zur enantioselektiven enzymatischen Reduktion einer Ketoverbindung zur entsprechenden chiralen Hydroxyverbindung.

Die erfindungsgemäßen Oxidoreduktasen sind dadurch gekennzeichnet, daß sie eine Aminosäuresequenz aufweisen, bei welcher mindestens 70% der Aminosäuren identisch sind mit den Aminosäuren der Aminosäuresequenz SEQ ID No 6.

Das Polypeptid entsprechend SEQ ID No 6 kann aus Bakterien der Gruppe Actinobacteria , insbesondere aus Bakterien der Gruppe Gordonia, insbesondere aus Gordonia rubripertincta DSMZ 43570 gewonnen werden. Gegenstand der Erfindung ist weiterhin eine Nukleinsäuresequenz SEQ ID No 14, die für das Protein mit der Aminosäuresequenz SEQ ID No 6 codiert. Das Polypeptid eignet sich sehr gut zur Reduktion von 2-Octanone zu S-2-Oktanol und oxydiert von beiden Enantiomeren des 2-Oktanol bevorzugt S-2-Oktanol oxidiert. Es eignet sich auch sehr gut zur Reduktion von 4-Haloacetoacetat ester zu R-4-Halo-3-hydroxybuttersäureestem.

Die Oxidoreduktase aus Gordonia rubripertincta DSMZ 43570 ist ein Homomer mit einem Molekulargewicht bestimmt im SDS-Gel von 41 $\pm$ 3 k Da. Das pH-Optimum für die Reduktionsreaktion liegt für diese Oxidoreduktase im Bereich von 4,5 bis 5,5 und das pH-Optimum für die Oxidationsreaktion liegt im Bereich von 7,5 bis 9,5. Die Oxidoreduktase aus Gordonia rubripertincta DSMZ 43570 weist eine gute Temperatur, pH- und Lösungsmittelstabilität auf und zeigt im pH-Bereich von 4,5-10 sowie bei Temperaturen bis 55 °C auch bei Inkubationszeiten von mehreren Stunden nur geringen Aktivitätsverlust.

Die Erfindung betrifft weiterhin Fusionsproteine, die dadurch gekennzeichnet sind, dass sie Oxidoreduktasen mit der Aminosäuresequenz SEQ ID No 6 oder deren Homologen darstellen, die mit einem weiteren Polypeptid am N-Terminalen oder Carboxy-terminalen Ende peptidisch verbunden sind. Fusionsproteine können beispielsweise leichter von anderen Proteinen abgetrennt werden oder können in größeren Mengen rekombinant exprimiert werden.

Die Erfindung betrifft ferner Antikörper, die spezifisch an die Oxidoreduktasen gemäß SEQ ID No 6 oder deren Homologen binden. Die Herstellung dieser Antikörper erfolgt nach bekannten Methoden durch Immunisierung von geeigneten Säugetieren und anschließender Gewinnung der Antikörper. Die Antikörper können monoklonal oder polyklonal sein.

Vergleiche von Aminosäuresequenzen können beispielsweise im Internet in Proteindatenbaken wie z.B. SWISS-PROT, PIR sowie in DNA-Datenbanken wie z.B.. EMBL, GenBank etc unter Nutzung des FASTA-Programm oder BLAST-Programm durchgeführt werden.

Das optimale Alignement wird dabei mit Hilfe des BLAST -Algorithmus (Basic Local Alignement Search Tool) ermittelt (Altschul et al. 1990, Proc. Natl. Acd Sci. USA. 87: 2264-2268*).* Als Scoring-Matrix zur Berechnung der Sequenzähnlichkeit wird die PAM30-Matrix zugrunde gelegt. (Dayhoff; M.O., Schwarz. R.M., Orcutt, B.C. 1978. "A model of evolutionary change in Proteins in "Atlas of Protein Sequence and structure" 5(3) M.O. Dayhoff (ed) 345-352, National Biomedical Research foundation*).*

Die Erfindung betrifft ebenso Proteinfragmente, die dadurch gekennzeichnet sind, dass sie Fragmente der Aminosäuresequenz SEQ ID No 6 darstellen, mit einer Anzahl von mehr als 26 Aminosäuren je Fragment.

Gegenstand der Erfindung ist weiterhin eine mikrobielle Carbonyldehydrogenase die N-terminal die Aminosäuresequenz MKAIQIIQ (SEQ ID No 56) und/oder C-terminal die Aminosäuresequenz DLRGRAVVVP (SEQ ID No 57) und/ oder eine der internen Partialsequenzen TAAGACHSD (SEQ ID No 58), TPYHAIKPSLP(SEQ ID No 59), DFVGLQPT (SEQ ID No 60), VYGAWGCG (SEQ ID No 61), DDARHLVP (SEQ ID No 62), MTLGHEGA (SEQ ID No 63) oder GGLGHVGIQLLRHL (SEQ ID No 64) enthält.

Die Erfindung betrifft ferner einen Klonierungsvektor, enthaltend eine oder mehrere Nukleinsäuresequenzen codierend für die Carbonylreduktasen gemäß SEQ ID No 6, oder deren Homologen. Die Erfindung umfasst des weiteren einen solchen Klonierungsvektor der zusätzlich zur Carbonylreduktase ein geeignetes Enzym zur Regenerierung des NAD(P) enthält, wie z.B. Formatdehydrogenasen, Alkoholdehydrogenasen oder Glucosedehydrogenase.

Die Erfindung betrifft weiterhin einen Expressionsvektor, der sich in einer Bakterien., Insekten-, Pflanzen- oder Säugetierzelle befindet und eine Nukleinsäuresequenz enthält die für die Carbonylreduktasen gemäß SEQ ID No 6 oder deren Homologen kodiert und die in geeigneter Weise mit einer Expressionskontrollsequenz verbunden ist. Die Erfindung betrifft ferner eine rekombinante Wirtszelle, die eine Bakterien-, Hefe, Insekten-, Pflanzen-, oder Säugetierzelle ist und mit einem solchen Expressionsvektor transformiert oder transfektiert wurde sowie ein Herstellungsverfahren zur Gewinnung einer Carbonylreduktase die auf Kultivierung einer solchen rekombinanten Wirtszelle beruht.

Geeignete Klonierungsvektoren sind beispielsweise ppCR-Script, pCMV-Script , pBluescript (Stratagene), pDrive cloning Vector (Quiagen, Hilden, Deutschland), pS Blue, pET Blue, pET LIC-Vektoren (Novagen, Madison, USA) sowie TA-PCR Klonierungsvektoren (Invitrogen, Karlsruhe, Deutschland).

Geeignete Expressionsvektoren sind beispielsweise pKK223-3, pTrc99a, pUC, pTZ, pSK, pBluescript, pGEM, pQE, pET, PHUB, pPLc, pKC30, pRM1/pRM9, pTrxFus,pAS1, pGEx, pMAL oder pTrx.

Geeignete Expressionskontrollsequenzen sind beispielsweise trp-lac (tac)-Promotor, trp-lac (trc) promotor, lac-Promotor, T7-Promotor oder λpL-Promotor.

Die Oxidoreduktasen gemäß SEQ ID No 6 oder deren Homologen lassen sich so gewinnen, dass die oben genannten rekombinanten E. coli Zellen kultiviert werden, die Expression der entsprechenden Oxidoreduktase induziert wird und anschließend nach etwa 10 bis 18 Stunden (h) die Zellen durch Ultraschallbehandlung, durch Naßvermahlung mit Glasperlen in einer Kugelmühle (Retsch, GmbH, Haan Deutschland 10 min, 24 Hz) oder mittels Hochdruckhomogenisator aufgeschlossen werden. Der erhaltene Zellextrakt kann entweder direkt verwendet werden oder weiter gereinigt werden. Dazu wird der Zellextrakt beispielsweise zentrifugiert und der erhaltene Überstand wird einer Ionenaustauschchromatographie unterworfen, beispielsweise durch Ionenaustauschchromatographie am Q-Sepharose Fast Flow ® (Pharmacia).

Die Erfindung betrifft ferner ein Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird, dadurch gekennzeichnet, daß eine Oxidoreduktase eingesetzt wird, die eine Aminosäuresequenz aufweisen, bei welcher mindestens 70% der Aminosäuren identisch sind mit den Aminosäuren der Aminosäuresequenz SEQ ID No 6.

Eine weitere bevorzugte Ausführungsform des erfindungsgemäßen Verfahrens besteht darin, daß die Ketoverbindung die allgemeine Formel I

$$R_1\text{-C(O)-}R_2 \qquad \text{(I)}$$

aufweist, in der R1 für einen der Reste

1) $-(C_1-C_{20})$-Alkyl, worin Alkyl geradkettig oder verzweigtkettig ist,
2) $-(C_2-C_{20})$-Alkenyl, worin Alkenyl geradkettig oder verzweigtkettig ist und gegebenenfalls bis zu vier Doppelbindungen enthält,
3) $-(C_2-C_{20})$-Alkinyl, worin Alkinyl geradkettig oder verzweigtkettig ist und gegebenenfalls bis zu vier Dreifachbindungen enthält,
4) $-(C_6-C_{14})$-Aryl,
5) $-(C_1-C_8)$-Alkyl-$(C_6-C_{14})$-Aryl,
6) $-(C_5-C_{14})$-Heterocyclus, der unsubstituiert oder ein-, zwei- oder dreifach durch -OH, Halogen, $-NO_2$ und/oder $-NH_2$ substituiert ist, oder
7) $-(C_3-C_7)$-Cycloalkyl,
steht, wobei die oben unter 1) bis 7) genannten Reste unsubstituiert sind oder unabhängig voneinander ein-, zwei- oder dreifach durch -OH, Halogen, $-NO_2$ und/oder $-NH_2$ substituiert sind,
und $R_2$ für einen der Reste
8) $-(C_1-C_6)$-Alkyl, worin Alkyl geradkettig oder verzweigtkettig ist,
9) $-(C_2-C_6)$-Alkenyl, worin Alkenyl geradkettig oder verzweigtkettig ist und gegebenenfalls bis zu drei Doppelbindungen enthält,
10) $-(C_2-C_6)$-Alkinyl, worin Alkinyl geradkettig oder verzweigtkettig ist und gegebenenfalls zwei Dreifachbindungen enthält, oder
11) $-(C_1-C_{10})$-Alkyl-C(O)-O-$(C_1-C_6)$-Alkyl, worin Alkyl gerade oder verzweigtkettig ist und unsubstituiert ist oder ein-, zwei- oder dreifach durch -OH, Halogen, $-NO_2$ und/oder $-NH_2$ substituiert ist,

steht, wobei die oben unter 8) bis 11) genannten Reste unsubstituiert sind oder unabhängig voneinander ein-, zwei- oder dreifach durch -OH, Halogen, $-NO_2$ und/oder
$-NH_2$ substituiert sind,
stehen.

Die Erfindung betrifft ferner ein Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird, das dadurch gekennzeichnet, daß eine Oxidoreduktase eingesetzt wird, für welche

(a) die Nukleinsäuresequenz SEQ ID No 14 codiert, oder für welche
(b) eine Nukleinsäuresequenz codiert, deren komplementärer Strang mit der in (a) genannten Nukleinsäureseqenz unter hochstringenten Bedingungen hybridisiert.

Unter dem Begriff Aryl werden aromatische Kohlenstoffreste verstanden mit 6 bis 14 Kohlenstoffatomen im

Ring. -($C_6$-$C_{14}$)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-($C_1$-$C_{20}$)-Alkyl wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl. Unter dem Begriff "-Co-Alkyl" wird eine kovalente Bindung verstanden.

Unter dem Begriff "($C_3$-$C_7$)-Cycloalkyl" werden cyclische Kohlenwasserstoffreste verstanden wie Cyclopropyl, Cylobutyl, Cyclopentyl, Cyclohexyl oder Cycloheptyl.

Der Begriff "-($C_5$-$C_{14}$)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe -($C_5$-$C_{14}$)-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, -$CF_3$ oder - C(O)-O-($C_1$-$C_4$)-Alkyl substituiert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind beispielsweise Ethyl-4-chloracetoacetat, Methylacetoacetat, Ethyl-8-chloro-6-oxooctansäure, Ethyl-3-oxovaleriat, 4-Hydroxy-2-butanon, Ethyl-2-oxovaleriat, Ethyl-2-oxo-4-phenylbuttersäure, Ethylpyruvat, Ethylphenylglyoxylat, 1-Phenyl-2-propanon, 2-chloro-1-(3-chlorphenyl)ethan-1-on, Acetophenon, 2-Octanon, 3-Octanon, 2-Butanon, 1-[3,5-bis(trifluoromethyl)phenyl]ethan-1-one, 2,5-Hexandion, 1,4-Dichlor-2-butanon, Acetoxyaceton, Phenacylchlorid, Ethyl-4-bromoacetoacetat, 1,1-Dichloraceton, 1,1,3-Trichloraceton oder 1-Chloraceton.

Die Oxidoreduktasen können in dem erfindungsgemäßen Verfahren entweder vollständig gereinigt oder teilweise gereinigt eingesetzt werden oder mit Zellen, enthaltend die erfindungsgemäßen Oxidoreduktasen, durchgeführt werden. Die eingesetzten Zellen können dabei nativ, permeabilisiert oder lysiert vorliegen. Bevorzugt werden die klonierten Oxidoreduktasen gemäß SEQ ID No 6 bzw. deren Homologe eingesetzt.

Je kg umzusetzender Verbindung der Formel I werden 5 000 bis 10 Mio U Oxidoreduktase eingesetzt. (nach oben offen) Der Enzymeinheit 1 U entspricht dabei der Enzymmenge die benötigt wird um 1 $\mu$mol der Verbindung der Formel I je Minute (min) umzusetzen

Die enzymatische Reduktion selbst läuft unter milden Bedingungen ab, so dass die erzeugten Alkohole nicht weiterreagieren. Die erfindungsgemäßen Verfahren weisen eine hohe Standzeit und eine Enantiomerenreinheit von in der Regel mehr als 95% der hergestellten chiralen Alkohole und eine hohe Ausbeute bezogen auf die eingesetzte Menge an Ketoverbindungen auf.

Die Carbonylverbindung wird im erfindungsgemäßen Verfahren in einer Menge von 3% bis 50% bezogen auf das Gesamtvolumen eingesetzt, bevorzugt von 5% bis 40%, insbesondere von 10%- 30%.

Eine bevorzugte Ausführungsform der Erfindung ist weiterhin dadurch gekennzeichnet, dass das bei der Reduktion gebildete NAD oder NADP kontinuierlich mit einem Cosubstrat zu NADH bzw. NADPH reduziert wird.

Als Cosubstrat werden dabei bevorzugt primäre und sekundäre Alkohole, wie Ethanol, 2-Propanol, 2-Butanol, 2-Pentanol, 3-Pentanol, 4-Methyl-2-pentanol, 2-Heptanol, 2-Octanol oder Cyclohexanol eingesetzt.

Diese Cosubstrate werden mit Hilfe einer Oxidoreduktase und NAD bzw. NADP zu den entsprechenden Aldehyden oder Ketonen und NADH bzw. NADPH umgesetzt. Dadurch kommt es zur Regenerierung des NADH bzw. NADPH. Der Anteil des Cosubstrates für die Regenerierung beträgt dabei von 5 bis 95 Vol%, bezogen auf das Gesamtvolumen.

Zur Regenerierung des Cofactors kann zusätzlich eine Alkoholdehydrogenase zugesetzt werden. Geeignete NADH-abhängige Alkoholdehydrogenasen sind beispielsweise erhältlich aus Bäckerhefe, aus Candida boidinii, Candida parapsilosis oder Pichia capsulata. Geeignete NADPH-abhängige Alkoholdehydrogenasen kommen ferner vor in Lactobacillus brevis (DE 196 10 984 A1), Lactobacillus minor (DE 101 19 274), Pseudomonas (US 5,385,833) oder in Thermoanaerobium brockii. Geeignete Cosubstrate für diese Alkoholdehydrogenasen sind die bereits genannten sekundären Alkohole wie Ethanol, 2-Propanol (Isopropanol), 2-Butanol, 2-Pentanol, 4-Methyl-2-pentanol, 2-Octanol oder Cyclohexanol.

Ferner kann die Cofactorregenerierung beispielsweise auch mit mittels NAD- oder NADPabhängiger Formiat-Dehydrogenase (Tishkov et al., J. Biotechnol. Bioeng. [1999] 64, 187-193, Pilot-scale production and isolation of recombinant NAD and NADP specific Formate dehydrogenase) durchgeführt werden. Geeignete Cosubstrate der Formiat-Dehydrogenase sind beispielsweise Salze der Ameisensäure wie Ammoniumformiat, Natriumformiat oder Calciumformiat. Bevorzugt werden die erfindungsgemäßen Verfahren jedoch ohne eine solche zusätzliche Dehydrogenase durchgeführt, d.h. es findet eine substratgekoppelte Coenzymregenerierung statt.

Der wässrige Anteil des Reaktionsgemisches, in dem die enzymatische Reduktion abläuft enthält bevorzugt einen Puffer, beispielsweise Kaliumphosphat-, Tris/HCl- oder Triethanolamin-Puffer, mit einem pH-Weit von 5 bis 10, vorzugsweise ein pH-Wert von 6 bis 9. Der Puffer kann zusätzlich noch Ionen zur Stabilisierung oder Aktivierung der Enzyme enthalten, beispielsweise Zinkionen oder Magnesiumionen.

Die Temperatur beträgt während der Durchführung der erfindungsgemäßen Verfahren zweckmäßig von etwa 10°C bis 70°C, bevorzugt von 20°C bis 40°C.

In einer weiteren bevorzugten Ausführungsform der erfindungsgemäßen Verfahren wird die enzymatische Umsetzung in Anwesenheit eines mit Wasser nicht oder nur beschränkt mischbaren organischen Lösungsmittels durchgeführt. Dieses Lösungsmittel ist beispielsweise ein symmetrischer oder unsymmetrischer Di($C_1$-$C_6$)alkylether, ein geradkettiges oder verzweigtes Alkan oder Cycloalkan oder ein wasserunlöslicher sekundärer Alkohol, der zugleich das Cosubstrat darstellt. Die bevorzugten organischen Lösungsmittel sind beispielsweise Diethylether, tertiär-Butylmethylether, Diisopropylether, Dibutylether, Butylacetat, Heptan, Hexan, 2-Oktanol, 2-Heptanol, 4-Methyl-2-pentanol oder Cyclohexan. Das Lösungsmittel kann dabei auch gleichzeitig als Cosubstrat zu Cofaktorregenerierung dienen.

Der Reaktionsansatz besteht beim Einsatz wasserunlöslicher Lösungsmittel bzw. Cosubstrate aus einer wässrigen und einer organischen Phase. Das Substrat verteilt sich entsprechend seiner Löslichkeit zwischen organischer und wässriger Phase. Die organische Phase hat allgemein einen Anteil von 5 bis 95%, bevorzugt von 10 bis 90% bezogen auf das gesamte Reaktionsvolumen. Die zwei flüssigen Phasen werden bevorzugt mechanisch gemischt, so dass eine große Oberfläche zwischen ihnen erzeugt wird. Auch in dieser Ausführungsform kann das bei der enzymatischen Reduktion gebildete NAD bzw. NADP mit einem Cosubstrat, wie beschrieben, wieder zu NADH bzw. NADPH reduziert werden.

Die Konzentration des Cofaktors NADH bzw. NADPH in der wässrigen Phase beträgt allgemein 0,001 mM bis 1mM, insbesondere 0,01 mM bis 0,1 mM.

In den erfindungsgemäßen Verfahren kann noch ein Stabilisator der Oxidoreduktase/Dehydrogenase eingesetzt werden. Geeignete Stabilisatoren sind beispielsweise Glycerin, Sorbitol, 1,4 -DL-Dithiothreit (DTT) oder Dimethylsulfoxid (DMSO).

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Die Reaktionszeit beträgt von 1 Stunde bis 48 Stunden, insbesondere von 2 Stunden bis 24 Stunden.

Anschließend wird das Reaktionsgemisch aufgearbeitet. Dazu wird die wässrige Phase abgetrennt, die organische Phase wird filtriert. Die wässrige Phase kann gegebenenfalls noch einmal extrahiert werden und wie die organische Phase weiter aufgearbeitet werden. Danach wird gegebenenfalls das Lösungsmittel aus der filtrierten organischen Phase verdampft.

Die Erfindung betrifft ferner ein Verfahren zur Gewinnung von chiralen Hydroxyverbindung der Formel II,

R1-C(OH)-R2 (II)

wobei $R^1$ und $R^2$ wie oben definiert sind, das dadurch gekennzeichnet ist, dass man

a) ein Gemisch, enthaltend die racemische Verbindung der Formel II, in Anwesenheit einer der erfindungsgemäßen Oxidoreduktasen gemäß SEQ ID No 6 oder deren Homologen , NAD(P) und Wasser inkubiert, wobei ein Enantiomer der Hydroxyverbindung der Formel II zur entsprechenden Ketoverbindung und NAD(P)H umgesetzt wird, und
b) das verbliebene Enantiomer der Hydroxyverbindung der Formel II isoliert.

Bei Einsatz der Carbonylreduktasen gemäß SEQ ID No 6 erhält man dabei bevorzugt die entsprechenden chiralen R-Hydroxyverbindungen.

Die Reaktionsbedingungen sind im wesentlichen dieselben wie im obengenannten Verfahren zur enantiospezifischen Reduktion der Ketoverbindung der Formel I. Es wird jedoch statt einer enantioselektiven Reduktion der Ketoverbindung der Formel I aus dem racemischen Gemisch der Verbindung der Formel II nur ein Enantiomer der Hydroxyverbindung der Formel II enantioselektiv zur entsprechenden Ketoverbindung oxidiert. Dadurch verbleibt das entgegengesetzte Enantiomer der Hydroxyverbindung der Formel II und kann isoliert werden. Ferner wird im Verfahren anstelle der als Cosubstrate eingesetzten Alkohole wie Ethanol, 2-Propanol (Isopropanol), 2-Butanol, 2-Pentanol oder 2-Octanol, deren entsprechenden Ketone wie Aceton zur Regenerierung des NAD eingesetzt. Beispielsweise wird das Aceton und NAD(P)H mit der erfindungsgemäßen Oxidoreduktase oder einer zusätzlichen Dehydrogenase zu NAD und Isopropanol umgesetzt.

Bevorzugte Ausführungsformen der Erfindung werden mit den folgenden Beispiele noch näher erläutert.

*Beispiel 1:*

*Anzucht von Organismen und Sreening nach Carbonylreduktaseaktivität*

Der Stamm Gordonia rubripertincta DSMZ 43570 wurde auf einem Medium aus Hefeextrakt (4), Glucose (4), Malzextrakt (10) und $CaCO_3$ (2) bei 37°C und 160 Umdrehungen pro Minute (rpm) kultiviert.

Anschließend wurden 125 mg Zellen mit 800 µl Aufschlusspuffer (100 mM Trieethanolamin (TEA), pH = 7,0) resuspendiert, mit 1 g Glasperlen versetzt und 10 Minuten (min) bei 4°C in der Kugelmühle aufgeschlossen (Retsch). Der nach 2 min Zentrifugation bei 12000 rpm erhaltene Überstand (Lysat) wurde im folgenden Aktivitätsscreening und zur Bestimmung des Enatiomerenüberschusses (ee-Wert) eingesetzt. Als Substrate wurden verschiedene Ketone, wie 2-Butanone, 2-Oktanon, Ethyl-4-chloracetoacetat, Acetophenon oder Ethyl-2-oxo-4-phenylbuttersäure verwendet.

Ansatz fürs Aktivitätsscreening:

| | |
|---|---|
| 860 µl | 0,1 M $KH_2PO_4/K_2PO_4$pH = 7,0 1mM $MgCl_2$ |
| 20 µl | NADPH oder NADH (10 mM) |
| 20 µl | Lysat |
| 100 µl | Substrat (100 mM) |

Die Reaktion wurde 1 min bei 340 nm verfolgt.

Ansatz für ee-Wert Bestimmung:

20 µl Lysat
100 µl NADH oder NADPH (50 mM)
60 µl Substrat (100 mM)

Die Ansätze zur ee-Bestimmung wurden nach 24 Stunden (h) beispielsweise mit Chloroform extrahiert und mittels Gaschromatographie (GC) wurde der Enantiomerenüberschuss bestimmt. Der Enantiomerenüberschuss wird wie folgt berechnet:

$$ee(\%) = ((R\text{-Alkohol} - S\text{-Alkohol})/(R\text{-Alkohol} + S\text{-Alkohol})) \times 100.$$

Tabelle 1

| DSMZ Nr. | Mikrorganismus | Aktivität in U/g Zellen Wirtsorganismus | | | |
|---|---|---|---|---|---|
| | | 2-Butanon | | 2-Oktanon | |
| | | NADH | NADPH | NADH | NADPH |
| 43570 | Gordonia rubripertincta | 7,7 | ----- | 13 | ----- |

DSMZ steht für Deutschen Sammlung für Mikroorganismen und Zellkulturen, Mascheroder Weg 1b, 38124 Braunschweig. Definition der Enzymeinheiten: 1 U entspricht der Enzymmenge die benötigt wird um 1 µmol Substrat pro min umzusetzen.

*Beispiel 2:*

*Isolierung und Reinigung von NAD(P)H abhängigen mikrobiellen Oxidoreduktasen*

Zur Isolierung der NAD(P)H abhängigen mikrobiellen Oxidoreduktasen wurden die Mikrorganismen wie unter Beispiel 1 beschrieben kultiviert. Nach Erreichen der stationären Phase wurden die Zellen geerntet und durch Zentrifugation vom Medium getrennt. Die Enzymfreisetzung erfolgte durch Naßvermahlung mittels Glasperlen, kann aber auch durch andere Aufschlussmethoden erreicht werden. Dazu wurden beispielsweise 100 g Zellfeuchtmasse mit 400 ml Aufschlusspuffer (100 mM Trieethanolamin, 1 mM $MgCl_2$, pH =7,0) suspendiert und mittels einer French press homogenisiert.

Der nach Zentrifugation (7000 rpm) erhaltene Rohextrakt wurde dann mittels FPLC (fast protein liquid chromatography) weiter aufgereinigt.

Alle erfindungsgemäßen Oxidoreduktasen konnten durch unterschiedliche Kombinationen von Ionenaustauschchromatographie, beispielsweise an Q-Sepharose Fast Flow (Pharmacia) oder Uno Q (Biorad, München, Deutschland), hydrophober Interaktionschromatographie, beispielsweise an Octylsepharose Fast Flow oder Butylsepharose Fast Flow (Pharmacia), Keramik-Hydroxyapatit-Chromatographie und Gelpermataion aufgereinigt werden.

*Beispiel 3:*

*Bestimmung der N-terminalen Sequenz einer erfindungsgemäßen Oxidoreduktase*

Die Enzympräparationen nach Beispiel 2 wurden nach der Gelpermeation im 10% igen Natriumdodecylsulfat (SDS) Gel aufgetrennt und auf eine Polyvinyliden Diflourid-Membran (PVDF-Membran) übertragen.

Die auffällige Bande wurde einer N-terminalen Sequenzierung mittels Edman-Abbau (Procise 492 (PE-Biosystems) unterworfen.

*Beispiel 4:*

*Allgemeine Klonierungsstrategie einer enantioselektiven Alkoholdehydrogenase isoliert aus Hefen*

Chromosomale DNA wird nach der in "Molecular cloning" von Manniatis & Sambrook beschriebenen Methode extrahiert. Die resultierende Nukleinsäure dient als Matrize für die Polymerasen Kettenreaktion (PCR) mit degenerierten Primem. Dabei werden 5'-Primer von der Aminosäurensequenz (SEQ ID No. 66; 72; 80) und die 3'-Primer von der Aminosäuresequenz (SEQ ID No. 67; 73, 81) unter Einbeziehung des für Organismus spezifischen Genkodes abgeleitet (SEQ ID No. 68; 69; 74; 75; 82; 83).

Die Amplifizierung wird in PCR-Puffer [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 40 pMol je Primer und 2.5 U BioTherm Star Polymerase (Genecraft, Lüdingshausen, Deutschland)] durchgeführt. Nach einer Aktivierung der BioTherm Star Polymerase (8 min 95°C) und folgenden 45-50 Zyklen einer Touch-Down PCR wird die Reaktion auf 4°C abgekühlt und der gesamte PCR-Ansatz auf ein 1% Agarose Gel zur Analyse aufgetragen.

Das spezifische Fragment, das der Polymerase Kettenreaktion resultiert, wird in den TA-Kloningsvektor pCR2.1 (Invitrogen, Karlsruhe, Deutschland) ligiert und mit den Primern M13 rev (SEQ ID No 65) und M13 uni (SEQ ID No 128) mit Hilfe von ABI DNA Sequenzer sequenziert.

Die 5'- und 3'-terminale Bereiche der Gen kodierenden Sequenz werden mit Hilfe von RACE Methode (rapid amplification of cDNA ends) bestimmt. Basierend auf der Nukleinsäuresequenz des spezifischen Fragments werden Oligonukleotide für 3'-RACE und 5'-RACE konstruiert. Als Matrize für die Synthese vom ersten cDNA Strang mit Hilfe von 3'-RACE System (Invitrogen, Karlsruhe, Deutschland) dient aus den Zellen präparierte gesamte RNA. Es folgen eine Amplifikation und eine Reamplifikation des spezifischen cDNA mit Hilfe von 3'-RACE Oligonukleotiden (SEQ ID No. 76; 77; 84; 85). Anschließend wird der Ansatz zur Analyse auf ein 1% Agarose Gel aufgetragen. Das spezifische Fragment, das die fehlende 3'-flankierende Sequenzinformation trägt, wird isoliert in einen TA-Kloningsvektor pCR2.1 ligiert und sequenziert.

Die kodierenden und nicht kodierenden 5'-terminalen Sequenzen werden mit Hilfe von 5'-RACE System (Invitrogen) bestimmt. Dazu wird mit Hilfe von Oligo dT-Cellulose (NEB, Beverly, USA) mRNA aus der zuvor gewonnenen Gesamt-RNA angereichert und in die Synthese des ersten cDNA Stranges mit den genspezifischen Oligonukleotiden (SEQ ID No. 70; 71; 78; 79; 86; 87) angesetzt. Nachfolgende Amplifikation und Reamplifikation der spezifischen cDNA resultiert in einem Fragment, das zur Analyse in ein pCR2.1 TA-Kloningsvektor (Invitrogen) ligiert wird. Das das Fragment enthaltende Plasmid wird mit Hilfe eines ABI DNA Sequenzer analysiert. Somit werden die fehlende Sequenzinformationen über 5'-Ende des Gens gewonnen.

Basierend auf der Sequenz, die für das Volllängen-Gen kodiert werden spezifische Primer für eine nachfolgende Klonierung dieses DNA Abschnittes in ein passendes Expressionssystem konstruiert. Dafür werden beispielsweise 5'-Primer mit einer Erkennungssequenz für Nde I, bzw. mit einer Erkennungssequenz für Sph I, bzw. für BamHI und 3' Primer mit einer Erkennungssquenz für Hind III modifiziert (SEQ ID No. 89; 90; 91; 92; 93; 94; 95; 96).

Chromosomale DNA dient in nachfolgender PCR als Matrize. Der für die jeweilige Oxidoreduktase kodierende DNA Abschnitt wird mit Hilfe von *Platinum pfx* Polymerase (Invitrogen) amplifiziert. Das resultierende PCR-Produkt wird nach der Reinigung über ein 1% Agarose Gel mit entsprechenden DNA Endonukleasen behandelt und in das mit den gleichen Endonukleasen behandelten Rückgrad des pET21a Vektors (Novagen, Madison, USA), bzw. Rückgrad des pQE70 Vektors (Qiagen, Hilden, Deutschland) ligiert.

Das entstandene Expressionskonstrukt wird nach der Sequenzierung in Expressionsstamm BL21 Star (Invitrogen), bzw. RB791 (E.coli genetic stock, Yale, USA) gebracht.

*Beispiel 5:*

*Allgemeine Klonierungsstrategie einer enantioselektiven Oxidoreduktase isoliert aus Bakterien*

Genomische DNA wird nach der in "Molecular cloning" von Manniatis & Sambrook beschriebenen Methode extrahiert. Die resultierende Nukleinsäure dient als Matrize für die Polymerasen Kettenreaktion (PCR) mit degenerierten Primern. Dabei werden 5'-Primer von der Aminosäurensequenz (SEQ ID No 104; 112) und die 3'-Primer von der Aminosäuresequenz (SEQ ID No 105; 113) unter Einbeziehung des für Organismus spezifischen Genkodes abgeleitet (SEQ ID No 106; 107; 114; 115)

Die Amplifizierung wird in PCR-Buffer [67mM Tris-HCl (pH 8.3), 16 mM $(NH_4)_2SO_4$, 115 mM $MgCl_2$, 0.01% Tween 20], 0.2 mM Desoxy-Nukleotidtriphosphat Mix (dNTPs), 40 pMol je Primer und 2.5 U BioTherm Star Polymerase (Genecraft, Lüdingshausen, Deutschland)] durchgeführt. Nach einer Aktivierung der BioTherm Star Polymerase (8 min 95 °C) und folgenden 45-50 Zyklen einer Touch-Down PCR wird die Reaktion auf 4°C abgekühlt und der gesamte PCR-Ansatz auf ein 1% Agarose Gel zur Analyse aufgetragen.

Das spezifische Fragment, das der Polymerase Kettenreaktion resultiert, wird in den TA-Kloningsvektor pCR2.1 (Invitrogen, Karlsruhe, Deutschland) ligiert und mit den Primern M13 rev (SEQ ID No 65) und M13 uni (SEQ ID No 128) mit Hilfe eines ABI DNA Sequenzer sequenziert.

5'-und 3'-flankierende Bereiche der Gen kodierenden Sequenz werden mit Hilfe von inversen Polymerase Kettenreaktionsmethode (iPCR) bestimmt. Basierend auf der Nukleinsäuresequenz des spezifischen internen Fragments werden Oligonukleotide SEQ ID No 100; 101; 102; 103; 108; 109; 110; 111; 116; 117; 118; 119 konstruiert. Genomische DNA wird mit Hilfe einer Restriktionsendonuklease verdaut und in eine Religation eingesetzt, so dass kleinere DNA Abschnitte zirkularisieren. Dieses Religationsgemisch wird dann als Matrize für eine iPCR und Primer SEQ ID No 100; 102; 108; 110; 116; 118 verwendet. Das PCR-Signal wird durch anschließende Nest-PCR mit Primer SEQ ID No 101; 103; 109; 111; 117; 119 verstärkt. Das resultierende spezifische Fragment wird nach der Elution aus dem 1% Agarose Gel in das Vektor pCR2.1 (Invitrogen) ligiert.

Somit liefert die Sequenzierungsanalyse des Fragment enthaltenden Vektors pCR2.1 die fehlende Sequenzinformationen über 3'- und 5'-kodierende Bereiche des Alkoholdehydrogenase / Reduktase Gens.

| Protein | Gordonia rubropertincta |
|---|---|
| Ansequenzierte Peptide | MKAIQIIQPG (SEQ ID No 112)<br>VGFFTQPYEVSVR (SEQ ID No 113) |
| Primer für Touch-Down PCR | ATGAARGCNATYCARATYA TYCARCC (SEQ ID No 114)<br>CYTCRTANGGYTGNGTRAA RAA (SEQ ID No 115) |
| Primer für iPCR | GAGGACGAAGTCGTCCGA ATG (SEQ ID No 116)<br>GCCGTCACCTTCAGCAACA CC (SEQ ID No 117)<br>CTCGACGTGAGCGACGACA AG (SEQ ID No 118)<br>GCAAGATCACCGGCAACG ATG (SEQ ID No 119) |

Basierend auf der Sequenz, die für das Volllängen-Gen kodiert (SEQ ID No.14) werden spezifische Primer für eine nachfolgende Klonierung dieses DNA Abschnittes in ein passendes Expressionssystem konstruiert. Dabei werden 5'-Primer mit einer Erkennungssequenz für Nde I, bzw. mit einer Erkennungssequenz für Sph I, bzw. für BamHI und 3' Primer mit einer Erkennungssquenz für Hind III modifiziert (SEQ ID No. 120; 121; 122; 123; 124; 125; 126; 127).

Die Amplifizierung der für das Protein kodierenden Volllängen-DNA aus genomischer DNA mit nachfolgender Restriktion und Ligation in den Expressionsvektor erfolgt wie im Beispiel 3 beschrieben. Der Expressionsstamm BL21 Star (Invitrogen), bzw. RB791 (E.coli genetic stock, Yale, USA) wird mit dem entstandenen Expressionskonstrukt transformiert.

*Beispiel 6:*

*Expression von rekombinanten Alkoholdehydrogenasen / Reduktasen in E.coli*

Die mit dem Expressionskonstrukt transformierte Escherichia coli Stämme BL21 Star (Invitrogen, Karlsruhe, Deutschland), bzw. RB791 (E.coli genteic stock, Yale, USA) wurden in 200 ml LB-Medium (1% Tryptone, 0.5% Hefeextrakt, 1% NaCl) mit Ampicillin (50 μg / ml), bzw. Carbenicillin (50 μg / ml) kultiviert, bis eine Optische Dichte gemessen bei 550 nm von 0.5 erreicht wurde. Die Expression von rekombinantem Protein wurde durch Zugabe von Isopropylthiogalaktosid (IPTG) in einer Konzentration von 0.1 mM induziert. Nach 8 Stunden, bzw. nach 16 Stunden Induktion bei 25 ° C und 220 rpm wurden die Zellen geerntet und bei -20 ° C eingefroren. Für den Aktivitätstest wurden 10 mg Zellen mit 500 μl 100 mM TEA Puffer pH 7.0 und 500 μl Glasperlen versetzt und 10 min mittels einer Kugelmühle aufgeschlossen. Das erhaltene Lysat wurde dann verdünnt für die entsprechenden Messungen eingesetzt. Aktivitätstest setzte sich wie folgt zusammen: 870 μl 100 mM TEA Puffer pH 7.0, 160 μg NAD(P)H, 10 μl verdünntes Zelllysat. Reaktion wurde gestartet bei der Zugabe von 100 μl einer 100 mM Substratlösung zum dem Reaktionsgemisch.

| | Expressionsvektor | Expressionsstamm | Substrat | Aktivität U / g |
|---|---|---|---|---|
| SEQ ID No 6 | pQE70 | RB791 | 2-Octanon | 1600 U/g |

*Beispiel 7:*

*Charakterisierung der rekombinanten Oxidoreduktasen*

*7a: pH-Optimum*

Es wurden die in Tabelle 4 aufgeführten Puffer hergestellt. Die Konzentration der jeweiligen Pufferkomponenten betrug jeweils 50 mM.

Tabelle 4

| PH-Wert | Puffersystem | pH-Wert | Puffersystem |
|---|---|---|---|
| 4 | Na-acetat/Essigsäure | 7,5 | $KH_2PO_4/K_2PO_4$ |
| 4,5 | Na-acetat/Essigsäure | 8 | $KH_2PO_4/K_2PO_4$ |
| 5 | Na-acetat/Essigsäure | 8,5 | $KH_2PO_4/K_2PO_4$ |
| 5,5 | $KH_2PO_4K_2PO_4$ | 9 | Glycin/NaOH |
| 6 | $KH_2PO_4/K_2PO_4$ | 9,5 | Glycin/NaOH |
| 6,5 | $KH_2PO_4/K_2PO_4$ | 10 | Glycin/NaOH |
| 7 | $KH_2PO_4/K_2PO_4$ | 11 | Glycin/NaOH |

Meßansatz (30°C)-pH Optimum Reduktion:

| | | |
|---|---|---|
| 870 | μl | der jeweils in Tabelle 3 genannten Puffersysteme |
| 20 | μl | NAD(P)H 10 mM |
| 10 | μl | Enzym verdünnt |

Es wurde etwa 2 bis 3 min inkubiert, danach erfolgte die Zugabe von

100 μl Substratlösung (100mM)

Als Substrat wurden je nach Oxidoreduktase 2-Butanon oder 2-Oktanon eingesetzt.
Die Reaktion wurde 1 min bei 340 nm verfolgt. Zur Bestimmung des pH-Optimums wurde die enzymatische Reaktion in dem jeweiligen in Tabelle 4 aufgeführten Puffer bestimmt. Zur Bestimmung des pH-Optimums für die Oxidationsreaktion wurde als Cofaktor NAD(P) und als Substrat 2-Propanol oder 2-Oktanol eingesetzt.

In Tabelle 5 sind die Ergebnisse für die erfmdungsgemäßen Oxidoreduktasen zusammengestellt.

Tabelle 5:

| DSMZ Nr. | Mikrorganismus | pH-opt red | pH-opt ox |
|---|---|---|---|
| 43570 | Gordonia rubripertincta | 5 | 7,5-9,5 |

*7b: pH-Stabilität*

Die Bestimmung der Aktivität der rekombinanten Oxidoreduktasen wurde durch Lagerung in den in Tabelle 4 genannten Puffersystemen untersucht. Dazu wurden die verschiedene Puffer (50mM) im Bereich von pH 4 bis 11 angesetzt und die gemäß Beispiel 4 hergestellte Oxidoreduktase damit verdünnt. Nach 30, 60 und 120 min Inkubation wurden aus dem Ansatz 10 μl entnommen und im Aktivitätstest gemäß Beispiel 1 eingesetzt.

Ausgangswert ist dabei der Messwert, den man unmittelbar nach Verdünnung (1:20) des Enzyms in Kaliumphosphatpuffer 50 mM pH = 7.0 erhielt. Dieser Wert entsprach unter den vorgegeben Bedingungen einer Extinktionsänderung von etwa 0,70 /min und wurde als 100%-Wert gesetzt und alle folgenden Messwerte wurden zu diesem Wert ins Verhältnis gesetzt.

In Tabelle 6 sind für die erfindungsgemäßen Oxidoreduktasen die pH-Bereiche zusammengestellt indem die Enzyme bei 120 min Inkubation nicht weniger als 50% der Ausgangsaktivität aufwiesen.

Tabelle 6:

| DSMZ Nr. | Mikrorganismus | pH-Bereich Stabilität |
|---|---|---|
| 43570 | Gordonia rubripertincta | 4,5-10 |

*7c: Temperaturoptimum*

Zur Bestimmung der optimalen Testtemperatur wurde die Enzymaktivität für die erfindungsgemäßen Oxidoreduktasen im Temperaturbereich von 15°C bis 70°C im Standardmeßansatz gemessen.

Die ermittelten Temperaturoptima sind in Tabelle 7 zusammengefasst:

Tabelle 7

| DSMZ Nr. | Mikrorganismus | Topt |
|---|---|---|
| 43570 | Gordonia rubripertincta | 45-55 °C |

*7d: Temperaturstabilität*

In analoger Weise wie unter Beispiel 5c beschrieben wurde die Temperaturstabilität für den Bereich von 15°C bis 70°C bestimmt. Dazu wurde jeweils eine Verdünnung der erfindungsgemäßen Oxidoreduktasen für 60 min und 180 min bei der jeweiligen Temperatur inkubiert und anschließend bei 30°C mit dem obigen Testansatz gemessen. In Tabelle 8 sind für die erfindungsgemäßen Oxidoreduktasen die Temperaturbereiche zusammengestellt indem die Enzyme bei 120 min Inkubation nicht weniger als 50% der Ausgangsaktivität aufwiesen.

Tabelle 8

| DSMZ Nr. | Mikrorganismus | Temperaturstabilität |
|---|---|---|
| 43570 | Gordonia rubripertincta | 15- 55 °C |

*7e: Substratspektrum*

Das Substratspektrum der erfindungsgemäßen Oxidoreduktasen wurde durch Messung der Enzymaktivität für Reduktion und Oxidation mit einer Reihe von Ketonen und Alkoholen bestimmt. Dazu wurde der Standard Messansatz gemäß Beispiel 1 mit unterschiedlichen Substraten verwendet.

Die Aktivität mit Methylacetoacetat wurde für alle Enzyme gleich 100% gesetzt und alle anderen Substrate wurden dazu ins Verhältnis gesetzt.

Tabelle 9: Substratsnektren Reduktion

| Substrat | Gordonia rubri-pertincta SEQID NO6 |
|---|---|
| 1-Phenyl-2-propanon | 82% |
| Phenacyl-Chlorid | 7 % |
| Acetophenon | 23 % |
| Acetonaphton | 68 % |
| Butyrophenon | 0% |
| 2-Octanon | 75 % |
| 3-Octanon | 52 % |
| 2-Butanon | 14 % |
| Ethyl-2-oxovaleriat | 23 % |
| Ethyl-2-oxo-4-phenyl buttersäure | 18 % |
| Ethylpyruvat | 160 % |
| Ethylphenyl-glyoxylat | 11 % |
| Ethyl-4-chloro acotoacetat | 110 % |
| Methyl acetoacetat | 100 % |
| Ethyl-3-oxovaleriat | 56 % |
| Aceton | 7 % |

*7f: Stabilität im wässrig/organischen Zwei -Phasensystem*

Die Stabilität der neuartigen Oxidoreduktasen in wässrig/ organischen Zweiphasensystemen wurde untersucht, indem die in Beispiel 6 gewonnenen Lysate (aus rekombinanter Expression) in einem für die jeweilige Oxidoreduktase geeigneten, wässrigem Puffer verdünnt wurden (ca. 10 Units/ml Puffer). Zu der im Puffer verdünnten Oxidoreduktase wurde dann das selbe Volumen eines organischen, nicht wassermischbaren Lösungsmittels gegeben und der Ansatz bei Raumtemperatur unter ständiger Durchmischung (Thermomixer mit 170 rpm) inkubiert. Nach 24 h Inkubation wurden jeweils aus der wässrigen Phase 10 µl entnommen und für die Bestimmung der Enzymaktivität im Standardtestansatz (Kaliumphosphatpuffer (KPP) 100 mM, pH = 7.0, 0,2 mM NAD(P)H, 10 mM Substrat) eingesetzt. Auch hier wurde der Ausgangswert direkt nach Verdünnung im Puffer gleich 100% gesetzt und alle weiteren Werte zu diesem ins Verhältnis gesetzt.

Tabelle 9: Enzymaktivität nach 24 h Inkubation im wässrig organischen Zwei-Phasensystem

| System | Puffer | Butylacetat | Diethylether | MTBE | Diisopropyl-ether | Heptan | Cyclohexan |
|---|---|---|---|---|---|---|---|
| Gordonia rubripertincta SEQ ID No 6 | 100% | 80-100% | 80-100% | 80-100% | 80-100% | 80-100% | 80-100% |
| MTBE = Methyl-tert-butylether | | | | | | | |

Tabelle 10: Substratspektren Oxidation

| Substrat | Gordonia rubri-pertincta SEQID NO6 |
|---|---|
| S-2-Oktanol | 100 % |
| R-2-Oktanol | 0 % |
| S-2-Butanol | 45 % |

(fortgesetzt)

| Substrat | Gordonia rubri-pertincta SEQID NO6 |
|---|---|
| R-2-Butanol | 17 % |
| S-Phenyl-2-propanol | 0 % |
| R-Phenyl-2-propanol | 0 % |
| Ethyl-(S)-4-chloro-3-hydroxybutyrate | 0 % |
| Ethyl-(R)-4-chloro-3-hydroxybutyrate | 0% |
| 2-Propanol | 27 % |
| Cyclohexanol | 7 % |

## Patentansprüche

1. Verfahren zur enantioselektiven enzymatischen Reduktion einer Ketoverbindung zur entsprechenden chiralen Hydroxyverbindung, wobei die Ketoverbindung in Gegenwart eines Co-Faktors mit einer Oxidoreduktase reduziert wird,

**dadurch gekennzeichnet, dass**
eine Oxidoreduktase eingesetzt wird, welche ausgewählt ist aus der Gruppe, bestehend aus den Oxidoreduktasen, für welche

(a) die Nukleinsäuresequenz SEQ ID NO:14 codiert, oder
(b) eine Nukleinsäuresequenz codiert, deren komplementärer Strang mit der in (a) genannten Nukleinsäuresequenz unter hochstringenten Bedingungen hybridisiert.

2. Verfahren nach Anspruch 1, **dadurch gekennzeichnet, dass** die Ketoverbindung die allgemeine Formel I

$$R_1\text{-C(O)-}R_2 \qquad (I)$$

aufweist, in der $R_1$ für einen der Reste

1) $-(C_1-C_{20})$-Alkyl, worin Alkyl geradkettig oder verzweigtkettig ist,
2) $-(C_2-C_{20})$-Alkenyl, worin Alkenyl geradkettig oder verzweigtkettig ist und gegebenenfalls bis zu vier Doppelbindungen enthält,
3) $-(C_2-C_{20})$-Alkinyl, worin Alkinyl geradkettig oder verzweigtkettig ist und gegebenenfalls bis zu vier Dreifachbindungen enthält,
4) $-(C_6-C_{14})$-Aryl,
5) $-(C_1-C_8)$-Alkyl-$(C_6-C_{14})$-Aryl,
6) $-(C_5-C_{14})$-Heterocyclus, der unsubstituiert oder ein-, zwei- oder dreifach durch -OH, Halogen, $-NO_2$ und/oder $-NH_2$ substituiert ist, oder
7) $-(C_3-C_7)$-Cycloalkyl,
steht, wobei die oben unter 1) bis 7) genannten Reste unsubstituiert sind oder unabhängig voneinander ein-, zwei- oder dreifach durch -OH, Halogen, $-NO_2$ und/oder $-NH_2$ substituiert sind,
und $R_2$ für einen der Reste
8) $-(C_1-C_6)$-Alkyl, worin Alkyl geradkettig oder verzweigtkettig ist,
9) $-(C_2-C_6)$-Alkenyl, worin Alkenyl geradkettig oder verzweigtkettig ist und gegebenenfalls bis zu drei Doppelbindungen enthält,
10) $-(C_2-C_6)$-Alkinyl, worin Alkinyl geradkettig oder verzweigtkettig ist und gegebenenfalls zwei Dreifachbindungen enthält, oder
11) $-(C_1-C_{10})$-Alkyl-C(O)-O-$(C_1-C_6)$-Alkyl, worin Alkyl gerade oder verzweigtkettig ist und unsubstituiert ist oder ein-, zwei- oder dreifach durch -OH, Halogen, $-NO_2$ und/oder $-NH_2$

substituiert ist,
steht, wobei die oben unter 8) bis 11) genannten Reste unsubstituiert sind oder unabhängig voneinander ein-, zwei- oder dreifach durch -OH, Halogen, $-NO_2$ und/oder $-NH_2$ substituiert sind, stehen.

3. Klonierungsvektor, enthaltend eine oder mehrere Nukleinsäuresequenzen, welche für die Oxidoreduktase gemäß SEQ ID NO:6 codieren.

4. Expressionsvektor, der sich in einer Bakterien-, Insekten-, Pflanzen- oder Säugetierzelle in vitro befindet und eine Nukleinsäuresequenz enthält, welche für die Oxidoreduktase gemäß SEQ ID NO:6 codiert und mit einer Expressionskontrollsequenz verbunden ist.

5. Rekombinante Wirtszelle, die eine Bakterien-, Insekten-, Pflanzen- oder Säugetierzelle ist und mit einem Expressionsvektor gemäß Anspruch 4 transformiert oder transfektiert wurde.

6. Oxidoreduktase zur Verwendung in einem Verfahren gemäß Anspruch 1 oder 2, **dadurch gekennzeichnet, dass** sie von der Nukleinsäuresequenz codiert wird, die ausgewählt ist aus der Gruppe bestehend aus SEQ ID NO:14 und einer Nukleinsäuresequenz, deren komplementärer Strang mit der Sequenz SEQ ID NO:14 unter hochstringenten Bedingungen hybridisiert.

7. Verfahren zur enantioselektiven enzymatischen Reduktion einer Ketoverbindung zur entsprechenden chiralen Hydroxyverbindung, wobei die Ketoverbindung mit einer Oxidoreduktase in Gegenwart eines Co-Faktors reduziert wird, **dadurch gekennzeichnet, dass**
eine Oxidoreduktase eingesetzt wird, die eine Aminosäuresequenz aufweist, bei welcher mindestens 70% der Aminosäuren identisch sind mit den Aminosäuren der Aminosäuresequenz SEQ ID NO:6.

## Claims

1. A process for the enantioselective enzymatic reduction of a keto compound to the corresponding chiral hydroxy compound, wherein the keto compound is reduced with an oxidoreductase in the presence of a cofactor, **characterized in that** an oxidoreductase is used which is selected from the group consisting of the oxidoreductases which

   (a) are encoded by nucleic acid sequence SEQ ID NO:14, or which
   (b) are encoded by a nucleic acid sequence the complementary strand of which hybridizes with the nucleic acid sequence mentioned in (a) under highly stringent conditions.

2. The process according to claim 1, **characterized in that** the keto compound has the general formula I

   $$R_1\text{-C(O)-}R_2 \qquad (I)$$

   wherein $R_1$ stands for one of the moieties

   1) -($C_1$-$C_{20}$)-alkyl, wherein alkyl is linear-chain or branched,
   2) -($C_2$-$C_{20}$)-alkenyl, wherein alkenyl is linear-chain or branched and optionally contains up to four double bonds,
   3) -($C_2$-$C_{20}$)-alkynyl, wherein alkynyl is linear-chain or branched and optionally contains up to four triple bonds,
   4) -($C_6$-$C_{14}$) -aryl,
   5) -($C_1$-$C_8$) -alkyl- ($C_6$-$C_{14}$) -aryl,
   6) -($C_5$-$C_{14}$)-heterocycle which is unsubstituted or substituted one, two or three times by -OH, halogen, -$NO_2$ and/or -$NH_2$, or
   7) - ($C_3$-$C_7$)-cycloalkyl,
   wherein the moieties mentioned above under 1) to 7) are unsubstituted or substituted one, two or three times, independently of each other, by -OH, halogen, -$NO_2$ and/or - $NH_2$,
   and $R_2$ stands for one of the moieties
   8) -($C_1$-$C_6$)-alkyl, wherein alkyl is linear-chain or branched,
   9) -($C_2$-$C_6$)-alkenyl, wherein alkenyl is linear-chain or branched and optionally contains up to three double bonds,
   10) - ($C_2$-$C_6$) -alkynyl, wherein alkynyl is linear-chain or branched and optionally contains two triple bonds, or
   11) - ($C_1$-$C_{10}$) -alkyl-C (O) -O- ($C_1$-$C_6$) -alkyl, wherein alkyl is linear or branched and is unsubstituted or substituted one, two or three times by -OH, halogen, -$NO_2$ and/or -$NH_2$,
   wherein the moieties mentioned above under 8) to 11) are unsubstituted or substituted one, two or three times, independently of each other, by -OH, halogen, -$NO_2$ and/or - $NH_2$.

3. A cloning vector comprising one or several nucleic acid sequences coding for the oxidoreductase according to SEQ ID NO:6.

4. An expression vector located in vitro in a bacterial, insect, plant or mammalian cell and comprising a nucleic acid sequence which codes for the oxidoreductase according to SEQ ID NO:6 and is linked to an expression control sequence.

5. A recombinant host cell which is a bacterial, insect, plant or mammalian cell and has been transformed or transfected with an expression vector according to claim 4.

6. An oxidoreductase for use in a process according to claim 1 or 2, **characterized in that** it is encoded by the nucleic acid sequence which is selected from the group consisting of SEQ ID NO:14 and a nucleic acid sequence the complementary strand of which hybridizes with the sequence SEQ ID NO:14 under highly stringent conditions.

7. A process for the enantioselective enzymatic reduction of a keto compound to the corresponding chiral hydroxy compound, wherein the keto compound is reduced with an oxidoreductase in the presence of a cofactor, **characterized in that** an oxidoreductase is used which has an amino acid sequence which is identical to the amino acid sequence SEQ ID NO:6 by at least 70 %.

## Revendications

1. Procédé pour la réduction énantiosélective enzymatique d'un composé cétonique en un composé hydroxylé chiral correspondant, dans lequel le composé cétonique est réduit par une oxydoréductase en présence d'un co-facteur, **caractérisé en ce que**
on utilise une oxydoréductase, laquelle est choisie parmi le groupe consistant en les oxydoréductases, codées par

    a. la séquence d'acide nucléique SEQ ID NO : 14, ou
    b. une séquence d'acide nucléique dont le brin complémentaire est hybridé avec la séquence SEQ ID NO : 14 dans des conditions de stringence élevées.

2. Procédé selon la revendication 1, **caractérisé en ce que** le composé cétonique présente la formule générale I

$$R_1\text{-}C(O)\text{-}R_2 \qquad (1)$$

où $R_1$ désigne les restes

1. -($C_1$-$C_{20}$)-alkyle, où l'alkyle est à chaîne droite ou ramifiée,
2. -($C_2$-$C_{20}$)-alcényle, où l'alcényle est à chaîne droite ou ramifiée et peut éventuellement comporter jusqu'à quatre doubles liaisons,
3. -($C_2$-$C_{20}$)-alcynyle, où l'alcynyle est à chaîne droite ou ramifiée et peut éventuellement comporter jusqu'à quatre triples liaisons,
4. -($C_6$-$C_{14}$)-aryle,
5. -($C_1$-$C_8$)-alkyle-($C_6$-$C_{14}$)-aryle,
6. un hétérocycle-($C_5$-$C_{14}$)- non substitué ou substitué une, deux ou trois fois par-OH, un halogène,-$NO_2$ et/ou -$NH_2$, ou
7. -($C_3$-$C_7$)-cycloalkyle,

où les restes 1) à 7) définis ci-dessus sont non substitués ou substitués une, deux ou trois fois, indépendamment les uns des autres, par -OH, un halogène, -$NO_2$ et/ou $NH_2$.
et $R_2$ désigne les restes

8. -($C_1$-$C_6$)-alkyle, où l'alkyle est à chaîne droite ou ramifiée,
9. -($C_2$-$C_6$)-alcényle, où l'alcényle est à chaîne droite ou ramifiée et peut éventuellement comporter jusqu'à trois doubles liaisons,
10. -($C_2$-$C_6$)-alcynyle, où l'alcynyle est à chaîne droite ou ramifiée et peut éventuellement comporter jusqu'à deux triples liaisons,
11. -($C_1$-$C_{10}$)-alkyle-C(O)-O-($C_1$-$C_6$)-alkyle, où l'alkyle est à chaîne droite ou ramifiée, non substitué ou substitué une, deux ou trois fois par -OH, un halogène, -$NO_2$ et/ou -$NH_2$,

où les restes 8) à 11) définis ci-dessus sont non substitués ou substitués une, deux ou trois fois, indépendamment les uns des autres, par -OH, un halogène, -$NO_2$ et/ou $NH_2$.

3. Vecteur de clonage comprenant une ou plusieurs séquences d'acide nucléique, lesquelles codent pour l'oxydoréductase conformément à SEQ ID NO : 6.

4. Vecteur d'expression se trouvant in vitro dans les cellules des bactéries, insectes, plantes ou mammifères et comportant une séquence d'acide nucléique, laquelle code pour l'oxydoréductase conformément à SEQ ID NO : 6 et est liée à une séquence de contrôle d'expression.

5. Cellule hôte recombinante qui est une cellule de bactérie, d'insecte, de plante, de mammifère et est transformée ou transfectée avec un vecteur d'expression conformément à la revendication 4.

6. Oxydoréductase pour utilisation dans un procédé selon la revendication 1 ou 2, **caractérisée en ce qu'**elle est codée par la séquence d'acide nucléique choisie dans le groupe consistant en SEQ ID NO : 14 et une séquence d'acide nucléique dont le brin complémentaire est hybridé avec la séquence SEQ ID NO : 14 dans des conditions de stringence élevée.

7. Procédé pour la réduction énantiosélective enzymatique d'un composé cétonique en un composé hydroxylé chiral correspondant, dans lequel le composé cétonique est réduit par une oxydoréductase en présence d'un co-facteur,

**caractérisé en ce que**
on utilise une oxydoréductase, laquelle comporte une séquence d'acides aminés dans laquelle au moins 70% des acides aminés sont identiques aux acides aminés de la séquence d'acides aminés SEG ID NO : 6.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10119274
- DE 10327454
- DE 10337401
- DE 10300335
- US 5523223 A
- US 5763236 A
- US 5200335 A
- DE 19610984 A1
- US 05385833 A
- US 5385833 A
- WO 12612005 A


### In der Beschreibung aufgeführte Nicht-Patentliteratur


- *Enzyme Microb Technol.,* November 1993, vol. 15 (11), 950-8
- *Biosci. Biotechnol. Biochem.,* 1999, vol. 63 (10), 1721-1729
- *Appl Microbiol Biotechnol.,* 26. April 2003, vol. 62 (4), 380-6
- *J Org Chem.,* 24. Januar 2003, vol. 68 (2), 402-6
- *Acta Crystallogr D Biol Crystallogr.,* Dezember 2000, vol. 56, 1696-8
- *Appl Microbiol Biotechnol.,* 26. Juni 2002, vol. 59 (4-5), 483-7
- *J. Org. Chem.,* 1992, vol. 57, 1532
- **ALTSCHUL et al.** *Proc. Natl. Acd Sci. USA.,* 1990, vol. 87, 2264-2268
- A model of evolutionary change in Proteins. **DAYHOFF; M.O. ; SCHWARZ. R.M. ; ORCUTT, B.C.** Atlas of Protein Sequence and structure. National Biomedical Research foundation, 1978, vol. 5, 345-352
- **TISHKOV et al.** Pilot-scale production and isolation of recombinant NAD and NADP specific Formate dehydrogenase. *J. Biotechnol. Bioeng.,* 1999, vol. 64, 187-193